# EUROPÄISCHE PATENTSCHRIFT

(11) **EP 4 201 377 B1**
(45) Veröffentlichungstag und Bekanntmachung des Hinweises auf die Patenterteilung: **13.05.2026**
(21) Anmeldenummer: 23153052.8
(22) Anmeldetag: 18.09.2017
(51) Int. Cl.: A61F 2/01

(54) **EMBOLIESCHUTZVORRICHTUNG, VERFAHREN ZU DEREN FALTUNG UND FORMVORRICHTUNG**
EMBOLISM PROTECTION DEVICE, METHOD FOR FOLDING SAME, AND MOLDING DEVICE
DISPOSITIF DE PROTECTION EMBOLIQUE, PROCÉDÉ DE PLIAGE ASSOCIÉ ET DISPOSITIF DE MOULAGE

(30) Priorität: 28.10.2016 DE 102016012869
(43) Veröffentlichungstag der Anmeldung: 28.06.2023
(62) Teilanmeldung aus: 17781388.8
(73) Patentinhaber: Protembis GmbH, 52074 Aachen (DE)
(72) Erfinder: JIMENÉZ DÍAZ, Victor Alfonso, 36207 Vigo Pontevedra (ES); PFENNIG, Michael, 52072 Aachen (DE); RASMUS, Conrad, 10119 Berlin (DE); SCHUMACHER, Oliver, 52066 Aachen (DE); VON MANGOLDT, Karl, 52074 Aachen (DE)
(74) Vertreter: Sattler de Sousa e Brito, Clara

(56) Entgegenhaltungen:
- WO-A1-2015/177322
- US-A1- 2010 324 589
- US-A1- 2013 103 075
- US-A1- 2014 163 603

## Beschreibung

Die Erfindung betrifft eine Embolieschutzvorrichtung nach dem Oberbegriff von Anspruch 1, die verhindert, dass unerwünschte makroskopische Teilchen aus einer Blutbahn in eine oder mehrere Gefäßabzweigungen eines Hauptgefäßes, wie des Aortenbogens, gelangen. Die Erfindung betrifft auch eine Formvorrichtung zur Umformung der erfindungsgemäßen Embolieschutzvorrichtung, als auch ein Verfahren zum Falten und zum Entfalten der erfindungsgemäßen Embolieschutzvorrichtung mittels der Formvorrichtung.

Zerebrale Embolie ist eine bekannte Komplikation in der Herzchirurgie und in der interventionellen Kardiologie. Partikel können durch chirurgische oder interventionelle Eingriffe gelöst werden. Sie können in den Blutstrom gelangen und insbesondere im Gehirn eine Embolie auslösen. Bei einer zerebralen Embolie kann dies zu einem Schlaganfall führen oder sogar tödlich sein.

Embolieschutzvorrichtungen sind beispielsweise aus EP2859864 des Anmelders bekannt. Des weiteren offenbart WO 2015/177322 A1 eine Embolieschutzvorrichtung zur Zuführung in einen Aortenbogen.

Es ist Aufgabe der vorliegenden Erfindung, eine verbesserte Embolieschutzvorrichtung anzugeben, so dass in einfacher Art und Weise verhindert wird, dass unerwünschte makroskopischen Teilchen aus einer Blutbahn in eine oder mehrere Gefäßabzweigungen eines Hauptgefäßes gelangen.

Die erfindungsgemäße Lösung der Aufgabe erfolgt durch die Merkmale der unabhängigen Ansprüche. Vorteilhafte Weiterbildungen der Erfindung ergeben sich aus den Unteransprüchen.

Ein erster Aspekt der Erfindung betrifft eine Embolieschutzvorrichtung zur Zuführung in einen Aortenbogen, umfassend eine Filtereinheit, einen Rahmen und eine Zuführungseinheit, wobei die Filtereinheit am Rahmen angeordnet ist. Der Rahmen weist einen proximalen Bereich auf, der eine Proximalform umfasst, welche in einem Innenbereich des Rahmens angeordnet ist und mit der Zuführungseinheit verbunden ist, wobei die Proximalform einen ersten Teil und einen zweiten Teil umfasst, wobei der zweite Teil an einem Ende des ersten Teils ausgebildet ist. Der Innenbereich des Rahmens umfasst sowohl die Ebene, die vom Rahmen aufgespannt wird, als auch den Bereich oberhalb oder unterhalb dieser Ebene.

Durch die erfindungsgemäße Embolieschutzvorrichtung wird in vorteilhafter Weise eine Vorrichtung angegeben, die sich dadurch auszeichnet, dass durch die Verbindung der Proximalform und der Zuführungseinheit ein Federmechanismus geschaffen wird, der dafür sorgt, dass die Embolieschutzvorrichtung in der Aorta im Wesentlichen im distalen Bereich in Richtung Kopfgefäße an die Gefäßwand gedrückt wird. Mit der Embolieschutzvorrichtung werden unerwünschte makroskopische Teilchen im Wesentlichen deflektiert.

Der proximale Bereich wird durch das Zurückziehen der Zuführungseinheit vor dem Ostium der linken Arteria Subclavia positioniert. So wird eine stabile Lage im Aortenbogen realisiert. In einem alterativen Positionierungsszenario kann die Embolieschutzvorrichtung auch über die rechte Arteria Subclavia eingebracht werden. Der proximale Bereich wird hierbei durch das Zurückziehen der Zuführungseinheit vor dem Ostium des Truncus Brachiocephalicus positioniert.

Der Federmechanismus wird insbesondere durch die Geometrie der Proximalform ausgebildet. Bevorzugt ist der erste Teil der Proximalform unterhalb der Ebene des Rahmens angeordnet, insbesondere im Innenbereich des Rahmens. Der erste Teil ist vorteilhaft bogenförmig ausgebildet. Der zweite Teil der Proximalform ist bevorzugt oberhalb des Rahmens angeordnet, insbesondere im Innenbereich des Rahmens. Der zweite Teil ist vorteilhaft gerade ausgebildet. Der erste und der zweite Teil weisen bevorzugt einen Winkel zueinander und/oder zu der Ebene des Rahmens auf. Mit anderen Worten, zumindest der erste und/oder der zweite Teil können oberhalb oder unterhalb der Ebene des Rahmens angeordnet sein, wobei der Winkel zwischen dem ersten Teil und der Ebene des Rahmens unterschiedlich zum Winkel zwischen dem zweiten Teil und der Ebene des Rahmens ist, so dass der erste und der zweite Teil einen Winkel einschließen.

Durch die Zuführungseinheit kann die Proximalform unter Spannung gebracht werden, so dass sich die Federwirkung über die Proximalform auf den gesamten Rahmen der Embolieschutzvorrichtung überträgt. Durch diese Spannungsübertragung wird insbesondere der distale Bereich des Rahmens hochgeklappt.

Der Rahmen der Embolieschutzvorrichtung spannt eine zweidimensionale Ebene auf und geht im proximalen Bereich in eine Proximalform über, die vorteilhafterweise aus dieser Ebene nach unten oder nach oben ragen kann. Die im Inneren des Rahmens angeordnete und mit der Zuführungseinheit verbundene Proximalform schafft den Federmechanismus, durch den gewährleistet ist, dass der Rahmen mit der Filtereinheit so über einem oder mehreren Blutgefäßen fixiert werden kann, dass diese geschützt oder abgedeckt sind. Beim Aufspannen der Filtereinheit wirken Radialkräfte. Die Positionierung der Embolieschutzvorrichtung wird durch den Federmechanismus sowie durch die Zuführungseinheit ausgeführt. Des Weiteren wird eine haptische Rückmeldung beim Positionieren der Embolieschutzvorrichtung oder ein Widerstand beim Zurückziehen der Zuführungsvorrichtung erreicht, so dass dadurch die korrekte Position der Embolieschutzvorrichtung überprüft werden kann. Insbesondere wird dergestalt auch das Kopfgefäß abgedeckt und geschützt, durch welches die Embolieschutzvorrichtung eingebracht wird.

Durch die Geometrie des Rahmens, insbesondere der Proximalform und/oder der Distalform passt sich die Embolieschutzvorrichtung den anatomischen Bedingungen im Aortenbogen flexibel und unabhängig vom Zugangsweg an und bietet eine komplette Abdeckung aller Kopfgefäße.

Vorteilhalft ist der erste Teil und der zweite Teil der Proximalform im Innenbereich des Rahmens angeordnet. Insbesondere ist die Verbindungsstelle zwischen Proximalform und Zuführungseinheit im Innenbereich des Rahmens angeordnet, so dass die Abdeckung des Zugangsgefäßes gewährleistet ist. Mit anderen Worten, der proximale Bereich des Rahmens bzw. der Filtereinheit deckt das Ostium des Zugangsgefäßes ab und ragt weit über dieses hinaus. Gleichzeitig liegt der proximale Bereich des Rahmens bzw. der Filtereinheit an der Aortenwand an. Damit ist insbesondere eine Abdeckung des Zugangsgefäßes gewährleistet, wenn die Embolieschutzvorrichtung im Aortenbogen platziert ist.

Die erfindungsgemäße Embolieschutzvorrichtung, insbesondere der Rahmen und die an ihm angeordnete Filtereinheit kann vollständig gefaltet und entfaltet werden. Im gefalteten Zustand ist die Embolieschutzvorrichtung bevorzugt so dimensioniert, dass sie einen Durchmesser von im Wesentlichen 1,4-2,2 mm, insbesondere 1,7-1,8 mm aufweist. Die Embolieschutzvorrichtung weist drei Zustände auf: ein entfalteter Zustand, in dem die Embolieschutzvorrichtung in ihrer Grundform ist (Grundzustand), ein gefalteter Zustand, beispielweise in einem Katheter (gefalteter Zustand), und ein entfalteter Zustand (Platzierungszustand), wenn die Embolieschutzvorrichtung ihrer Bestimmung nach verwendet wird, beispielsweise im Aortenbogen in der Endposition. Die Endposition im Aortenbogen wird nachfolgend auch Platzierungsposition genannt.

Die Geometrieform der drei Zustände ist unterschiedlich. Während des Transports und der Vorbereitung zur Implantation befindet sich die Embolieschutzvorrichtung in ihrer Grundform, wie beispielsweise in den Figuren gezeigt. Durch mechanische Umformung wird die Grundform in den gefalteten Zustand überführt. Das reversibel verformbare Material des Rahmens, beispielsweise ein superelastischer Nitinoldraht, lässt sich so verformen, dass sich die Embolieschutzvorrichtung in einen Katheter schieben lässt. Die Embolieschutzvorrichtung streckt sich dabei längs ihrer Richtung, wobei sie durch Umklappen der Distalform und der Proximalform in einen Außenbereich des Rahmens in eine gerade oder gestreckte Form übergeht. Die damit einhergehende Längenänderung ist bedingt durch die Verringerung der Breite. Der gefaltete Rahmen, d.h. die zwei Seiten des Rahmens außerhalb der Distalform und/oder Proximalform, liegen dabei von der Spitze bis zum Ende des Rahmens, also von der Distalform bis zur Proximalform, parallel zueinander im Katheter. Die speziell befestigte Filtereinheit kann dieser mechanischen Verformung folgen und legt sich in den Zwischenraum zwischen Katheter und Draht. Der aus Nitinol gefertigte Rahmen weist einen sogenannten Shape Memory Effekt auf.

In der Platzierungsposition im Aortenbogen passt sich die Geometrie des Rahmens der Embolieschutzvorrichtung flexibel der Aortenwand an und liegt in einem leichten Bogen, der Krümmung der Aorta folgend, vor den Kopfgefäßabgängen. Beim Verlassen des Katheters klappt sowohl die Distalform als auch die Proximalform in ihre ursprüngliche Form zurück und ermöglicht so eine atraumatische Positionierung des Rahmens an der Aortenwand. Durch die spezielle Form der eingeklappten Distalform und Proximalform werden scharfkantige Übergänge oder Ecken vermieden. Radialkräfte die durch den Shape Memory Effekt des Rahmens erzeugt werden, spannen dabei die Filterfläche auf. Eine zusätzliche Stabilisierung des Rahmens wird durch die physiologischen Bedingungen in der Aorta erreicht, da der Blutfluss durch den Oberflächenwiderstand des Filters den Rahmen der Embolieschutzvorrichtung zusätzlich in seine Platzierungsposition drückt.

Das Material des Rahmens ist bevorzugt Nitinol. Der Rahmen kann ein Draht oder ein Hohldraht sein, in dessen Hohlraum ein Platin-/Platin-Iridium-/Tantaldraht eingelegt ist, wobei der Hohlraum nahezu vollständig ausgefüllt ist. Alternativ kann der Rahmen aus DFT Wire, beispielsweise der Fa. Fort Wayne Metals, oder ein Draht mit einem fest verbundenen Platin/Tantalkern sein. Diese Beispiele für das Material des Rahmens haben den Vorteil, dass der Rahmen röntgenopak sichtbar ist.

Die Filtereinheit umfasst ein Filtermaterial das selektiv durchlässig ist, so dass beispielsweise unerwünschte makroskopische Teilchen aus der Blutbahn nicht in eine oder mehrere Gefäßabzweigungen eines Hauptgefäßes, wie des Aortenbogens, gelangen. Beispielsweise umfasst das Filtermaterial verschiedene Materialien, wie Kunststoffe oder metallartige Materialien wie Nitinol. Es ist möglich, je nach verwendetem Material, das Filtermaterial zu weben, zu gießen, zu lasern oder zu stanzen. Bevorzugt ist das Filtermaterial eine gewobene Membran aus Polyamid. Das Filtermaterial weist vorzugsweise eine Porengröße von 40-150 µm und eine Offenporigkeit von 35-60% auf, wodurch zum einen ein guter Schutz vor unerwünschtem Partikeln und zum anderen eine gute Blutdurchlässigkeit gewährleistet ist. Das Filtermaterial kann rechteckige oder quadratische Lochflächen aufweisen. Die Dicke des Filtermaterials ist vorzugsweise 20-120 µm.

Die Zuführungseinheit ist eine Röhre aus gewickeltem Edelstahldraht, jedoch können auch andere Materialien gewählt werden. Die Zuführungseinheit ist knickstabil und dient beim Positionieren der Embolieschutzvorrichtung zur Drehkraft- und Kraftübertragung. Vorteilhafterweise beträgt die Länge der Zuführungseinheit 120-250 cm bei einem Durchmesser von 1,5 mm und weist außen eine Ummantelung aus Kunststoff (Pebax-Beschichtung, Polyethylen (PE), Polytetrafluorethylen (PTFE), Polyamid (PA)) auf.

Die Länge der Embolieschutzvorrichtung ist vorteilhafterweise 50 bis 100 mm. Die Breite der Embolieschutzvorrichtung ist vorteilhafterweise 15 bis 45 mm.

Die Embolieschutzvorrichtung sieht in einer vorteilhaften Weiterentwicklung vor, dass der erste Teil der Proximalform zur Ebene des Rahmens einen ersten Winkel aufweist und der zweite Teil zum ersten Teil der Proximalform einen zweiten Winkel aufweist. Vorteilhalft sind der erste und zweite Teil der Proximalform an der Verbindungsstelle zwischen erstem und zweitem Teil koaxial ausgerichtet und bilden den Federmechanismus aus, indem durch die Zuführungseinheit die Winkelgrößen verändert werden, so dass die Embolieschutzvorrichtung in der Platzierungsposition im Aortenbogen fixiert werden kann. Der erste Teil der Proximalform weist einen Winkel von etwa 25 bis 50 Grad, vorzugsweise 30 Grad, nach unten zur zweidimensionalen Ebene des Rahmens, gemessen vom ersten Teil zur Ebene hin. Der erste Teil ist gerade oder bogenförmig und weist vorzugsweise eine Länge von 0,5 bis 2,5cm cm auf. Am Ende des ersten Teils ist der zweite vorzugsweise gerade ausgeformte Teil angeordnet. Der zweite Teil schließt mit dem ersten Teil einen zweiten Winkel von vorzugsweise 80 bis 115 Grad, vom zweiten Teil zum ersten Teil hin gemessen. Wird der zweite Winkel zur zweidimensionalen Ebene des Rahmens hin gemessen, ist er im Wesentlichen 110 bis 145 Grad, gemessen vom zweiten Teil zur Ebene hin. Die Länge des zweiten Teils beträgt vorzugsweise im Wesentlichen 1-5 cm. Diese geometrische Form der Proximalform sorgt dafür, dass die Proximalform eine Geometrie im platzierten Zustand aufweist, die an die Anatomie angepasst ist.

Eine andere vorteilhafte Weiterentwicklung sieht vor, dass die Proximalform zwei Enden des Rahmens umfasst, die sich parallel zueinander im Innenbereich des Rahmens erstrecken. Dies führt zu einer erhöhten Stabilität des Rahmens in einer Längsrichtung als auch in einer Querrichtung des Rahmens. In Weiterentwicklungen der Embolieschutzvorrichtung sind die zwei Enden mittels einer Klebeverbindung mit der Zuführungseinheit verbunden. Die Drahtenden sind somit nicht frei zugänglich. Es sind auch Weiterbildungen möglich in denen die Proximalform nur ein Ende des Rahmens umfasst, wobei das zweite Ende des Rahmens beispielsweise mit der Zuführungsvorrichtung verbunden ist.

In vorteilhaften Weiterentwicklungen ist vorgesehen, dass der Rahmen einen distalen Bereich aufweist, der eine Distalform umfasst, welche in einem Innenbereich des Rahmens angeordnet ist. Vorteilhaft ist die Spitze der Distalform mit atraumatischem Material (bspw. Membranmaterial, Polymer, Gummi oder Hartkleber) umhüllt, so dass ein atraumatischer Schutz vorhanden ist. Dieses Material kann vorteilhaft als Nase in Tröpfchenform ausgebildet sein.

In einer anderen vorteilhaften Weiterentwicklung zeichnet sich die Distalform dadurch aus, dass sie eine zum Inneren des Rahmens hin gerichtete Einschnürung aufweist. Die Einschnürung dient als Anbindungsstelle für die Filtereinheit. Sie dient zusätzlich als Positionierungshilfe in der Aorta, da sie mit röntgenopaken Markern versehen ist und als Mittel für die Anzeige der Ausrichtung des Rahmens im Katheter vorteilhaft verwendet werden kann. Die im Innenbereich des Rahmens angeordnete Distalform dient des Weiteren in vorteilhafter Weise als Positionierungshilfe beim Einschub der Embolieschutzvorrichtung durch eine Formvorrichtung in einen Katheter. Dabei kann die Distalform an oder in der Formvorrichtung eingehakt und in die zur ursprünglichen Richtung entgegengesetzte Richtung umgeklappt werden. Mit anderen Worten, die Distalform kann nach außen, also in einen Bereich außerhalb des Innenbereiches des Rahmens umgeklappt werden. Dies hat den Vorteil, dass beim Durchschub durch beispielsweise einen Katheter der Rahmen platzsparend im Katheter angeordnet werden kann. Durch das Umklappen der Distalform werden Torsionskräfte auf den Rahmen übertragen, die zu einem Zurückklappen der Distalform in den Innenbereich des Rahmens beim Entfalten der Embolieschutzvorrichtung im Aortenbogen führen.

Erfindungsgemäß ist die Verbindung von Rahmen und Filtereinheit eine Klebetunnelverbindung. Die Klebetunnelverbindung ist als umhüllende Polymerform um den Rahmen ausgeführt. Mit anderen Worten, der Kleber umhüllt den Rahmen rohrartig oder zylinderförmig. Die Polymerform bildet einen sogenannten Klebetunnel aus, in dem der Rahmen angeordnet ist und sich relativ zu diesem bewegen kann. Die Verbindung des Klebetunnels und der Filtereinheit kann vorteilhafterweise auch mechanisch stabil sein. Durch die Separation der Filtereinheit vom Rahmen entsteht eine Flexibilität im distalen und proximalen Bereich, die das Umklappen dieser Bereiche beim Falten oder Entfalten der Embolieschutzvorrichtung ermöglicht oder zumindest erleichtert.

In einer anderen vorteilhaften Weiterentwicklung ist vorgesehen, dass die Filtereinheit außerhalb des proximalen und/oder distalen Bereichs mit dem Rahmen verbunden ist. Diese Verbindung ist mechanisch stabil, das heißt ohne Relativbewegung zwischen Verklebung und Rahmen. Vorteilhaft ist die Verbindung als flexible Verbindung ausgebildet. Beispielweise kann die Verbindung eine Verklebung, ein Formschluss, eine Verschweißung oder ein Vernähen sein. Der Klebetunnel ermöglicht eine stabile und flexible Verbindung auch oder insbesondere während des Faltens oder Entfaltens der Embolieschutzvorrichtung.

In einer weiteren vorteilhaften Weiterbildung ist die Filtereinheit im distalen Bereich im Wesentlichen bis zur Einschnürung mit dem Rahmen verbunden. Dies verhindert ein ungewolltes Umklappen der Filtereinheit unterhalb des Rahmens. Die Verbindung kann bis zum Anfang der Einschnürung ausgebildet sein.

In einer anderen vorteilhaften Weiterbildung ist die Filtereinheit im proximalen Bereich im Wesentlichen bis zum ersten Teil der Proximalform mit dem Rahmen verbunden. Die Verbindung kann bis zum Anfang des ersten Teils ausgebildet sein.

In einer anderen vorteilhaften Weiterentwicklung ist die Filtereinheit im distalen und proximalen Bereich flexibel mit dem Rahmen verbunden. Somit wird eine Relativbewegung zwischen Rahmen und Filtereinheit ermöglicht. Dadurch ist gewährleistet, dass der Rahmen im proximalen und distalen Bereich beweglich mit der Filtereinheit verbunden ist. Hingegen ist der Rahmen im restlichen Bereich unbeweglich mit der Filtereinheit verbunden.

Erfindungsgemäß ist diese Verbindung eine Klebetunnelverbindung, die bevorzugt als umhüllende Polymerform um den Rahmen ausgebildet ist. Für eine Klebetunnelverbindung wird der Rahmen in einen Kleber hineingelegt. Beim Trocknen des Klebers verschiebt sich der Kleber und bildet eine Polymerform auf der Filtereinheit (wie ein Rohr). Geometrieänderungen der Embolieschutzvorrichtung, insbesondere des Rahmens, beispielsweise beim Falten oder Entfalten des Rahmens bzw. der Filtereinheit, kann so in einfacher Art und Weise getätigt werden, sodass ein Verzug oder im äußersten Fall eine Beschädigung der Embolieschutzvorrichtung verhindert wird.

Bevorzugt ist der Rahmen in einer Vorspannung mit der Filtereinheit verbunden. Beispielsweise kann der Rahmen leicht zusammengedrückt mit der Filtereinheit verbunden werden, um so eine Vorspannung zu bewahren. Die Verbindung des Filtermaterials mit dem Rahmen ist bevorzugt so ausgebildet, dass der Rahmen im Grundzustand sowie im platzierten Zustand eine Vorspannung auf die Filtereinheit ausübt.

Vorteilhafterweise ist die Filtereinheit von der Unterseite an den Rahmen geklebt. Dies führt dazu, dass zum Blutstrom hin eine glatte Oberfläche erzeugt werden kann.

In einer vorteilhaften Weiterentwicklung wird der Rand der Filtereinheit vor dem Befestigen am Rahmen versiegelt, um Formveränderung während der Anwendung zu verhindern, sowie eine möglichst atraumatischen Interaktion mit der Aortenwand zu ermöglichen.

In einer anderen vorteilhaften Weiterentwicklung ist vorgesehen, dass die Filtereinheit einen Überstand über den Rahmen aufweist. Vorzugsweise weist die Filtereinheit den Überstand außerhalb des proximalen und/oder distalen Bereichs auf. Der Überstand der Filtereinheit über dem Rahmen außerhalb des distalen bzw. proximalen Bereichs ist vorteilhafterweise zwischen 0,5 und 2,0 mm breit, so dass eine Dichtungslippe zur Gefäßwand gebildet wird, wenn der Rahmen im Platzierungszustand im Aortenbogen positioniert ist. Die Dichtungslippe begünstigt eine atraumatische Platzierung des Rahmens sowie dessen Formstabilität. Zusätzlich schließt diese Dichtungslippe im Platzierungszustand mit der Aortenwand ab und verhindert damit, wie ein Ventil, einen Leckagefluss im Seitenbereich der Embolieschutzvorrichtung.

Vorteilhaft ist der Überstand versiegelt, wodurch ein glatter Abschluss Filtermaterials gegeben ist. Dies begünstigt zudem die atraumatische Platzierung des Rahmens.

In einer anderen vorteilhaften Weiterentwicklung ist vorgesehen, dass die Filtereinheit im proximalen und/oder distalen Bereich des Rahmens über den Rahmen von der Unterseite zur Oberseite hin umgelegt ist. Die Filtereinheit steht somit in den Außenbereich des Rahmens über. Durch das Umlegen oder Umschlagen/Umklappen der Filtereinheit über den proximalen und/oder distalen Bereich des Rahmens wird eine verbesserte Fixierung der Filtereinheit am Rahmen erreicht, wobei die vollständige Abdeckung aller Kopfgefäße gewährleistet ist, wenn die Embolieschutzvorrichtung im Aortenbogen positioniert ist. Die Filtereinheit liegt im proximalen und/oder distalen Bereich als Doppellage und erhöht somit die Filterwirkung.

In einer anderen vorteilhaften Weiterentwicklung wird die Filtereinheit im distalen und/oder proximalen Bereich mittels eines Fadens, Garns oder Drahtes an der Distalform bzw. Proximalform befestigt. Zusätzlich kann diese Verbindung versiegelt werden, um Formstabilität zu erreichen. Des Weiteren wirkt durch die Versiegelung der Verbindung, dieser Bereich atraumatisch.

In einer vorteilhaften Weiterentwicklung ist die Filtereinheit mittels einer Verklebung an der Distalform befestigt. Die Verklebung kann mittels eines Hartklebers erfolgen. Durch die Verklebung weist der Rahmen eine atraumatische Spitze im distalen Bereich, auf, so dass bei einer Berührung des distalen Bereichs mit beispielsweise der Aortenwand, diese vor Verletzungen geschützt ist.

Vorteilhaft ist die Filtereinheit mittels einer Wendel im proximalen Bereich befestigt. Die Wendel ist als Edelstahldraht ausgebildet, der die Enden des Rahmens vorzugsweise spiralförmig umwickelt. Die Wendel dient der Stabilisierung der Verbindung zwischen Filtereinheit und Zuführungseinheit. Zudem begünstigt die Ausbildung als Wendel die Formänderung während des Faltens und Entfaltens der Embolieschutzvorrichtung.

Um die Embolieschutzvorrichtung noch weiter zu verbessern, ist in einer vorteilhaften Weiterentwicklung vorgesehen, dass die Filtereinheit ein Fasermaterial aufweist, wobei die Fasern so ausgerichtet sind, dass sie einen Winkel von im Wesentlichen 45 Grad zu einer Längsachse des Rahmens aufweisen. Das Fasermaterial besteht aus einer gewobenen Membran, wodurch eine erhöhte Flexibilität in Längs- und Querrichtung des Rahmens gewährleistet ist. Die Längsrichtung des Rahmens erstreckt sich vom proximalen zum distalen Bereich und ist bevorzugt die Mittellinie des Rahmens. Vorzugsweise bildet die Schräglage der Fasern einen Winkel von 45 ± 10 Grad zur Längsachse des Rahmens.

In einer anderen vorteilhaften Weiterentwicklung ist vorgesehen, dass die Proximalform mit der Zuführungseinheit verbunden ist, wobei die zwei Enden des Rahmens von einem Draht umwickelt sind, dessen Enden parallel zu den Enden des Rahmens angeordnet sind. Die Anbindung der Proximalform des Rahmens an die Zuführungseinheit erfolgt bevorzugt über eine Klebeanbindung. Die Enden des Rahmens sind in ein offenes Lumen der Zuführungseinheit eingeschoben und dort verklebt. Der Draht, der die Enden des Rahmens umwickelt und dadurch fixiert, ist vorzugsweise ein Edelstahldraht und dient der zusätzlichen Stabilisierung der Enden des Rahmens. Die Enden des umgewickelten Edelstahldrahtes liegen parallel zu den Enden des Rahmendrahtes und sind vorzugsweise zusammen in der Zuführungseinheit verklebt. Der Übergang vom Rahmendraht zur Zuführungseinheit, sowie der umgewickelte Edelstahldraht sind bevorzugt flexibel versiegelt, bspw. mit einer Polymermischung, um eine glatte Oberfläche sowie einen gleichmäßigen Übergang zu ermöglichen.

Bevorzugt weist der Rahmen der Embolieschutzvorrichtung eine Grundform auf, die als ovale Form ausgebildet ist. Die ovale Form ist der nativen Form des Aortenbogendaches angepasst und ermöglicht so die zuverlässige Abdeckung aller drei Kopfgefäße. Der obere Bereich des Aortenbogens an dieser Stelle ist wie z.B. das Innere einer auf dem Kopf stehenden ovalen Schüssel ausgeformt. Durch das Einlegen einer ovalen Form erreicht man so einen Formschluss. Bevorzugt verjüngt sich die ovale Form zur Proximalform hin. Mit anderen Worten, der Querschnitt durch die Aorta an der Stelle, an die die Embolieschutzvorrichtung platziert wird, ist oval, so dass sich die ovale Form des Rahmens vorteilhaft an die physiologische Form an dieser Stelle anpasst.

Ein zweiter Aspekt der Erfindung betrifft eine Formvorrichtung zur Umformung der erfindungsgemäßen Embolieschutzvorrichtung. Die Embolieschutzvorrichtung weist alle oder zumindest einige der genannten Merkmale auf - diese werden an dieser Stelle nicht erneut einzeln wiedergegeben. Die Umformung erfolgt zum Zuführen der Embolieschutzvorrichtung in einen Katheter, wobei der Rahmen mit der daran angeordneten Filtereinheit der Embolieschutzvorrichtung von einem expandierten Zustand in einen gestreckten Zustand umgeformt wird. Die Formvorrichtung weißt zwei Teilbereiche auf, die sich in einem engsten Querschnitt treffen. Die Teilbereiche sind jeweils vorzugsweise trichterförmig ausgebildet. Der distale Teilbereich ist bevorzugt als flacher oder runder Trichter ausgebildet und dient zum Einzug der erfindungsgemäßen Embolieschutzvorrichtung. Der proximale Teilbereich ist bevorzugt als kreisrunder Trichter ausgebildet und dient zur Aufnahme eines im Wesentlichen kreisrunden Schlauches, z.B. einer kommerziell erhältlichen Einführschleuse oder eines kommerziell erhältlichen Katheters. Die Formvorrichtung, ist ein Werkzeug mit einer Geometrie, die es erlaubt die Embolieschutzvorrichtung, insbesondere deren Rahmen so umzuformen, dass ihr Durchmesser im gefalteten Zustand bevorzugt im Wesentlichen 1,4-2,0 mm, insbesondere 1,7-1,8 mm aufweist. Mit der Formvorrichtung ist gewährleistet, dass die Embolieschutzvorrichtung in einfacher Art und Weise in einen im Wesentlichen kreisrunden Schlauch, z.B. eine kommerziell erhältliche Einführschleuse oder einen kommerziell erhältlichen Katheter eingezogen werden kann.

In einer Weiterentwicklung ist die bevorzugte flache oder runde Öffnung der Formvorrichtung so ausgebildet, dass die Proximalform und/oder eine Distalform des Rahmens der Embolieschutzvorrichtung nach außen geklappt werden. Dies gewährleistet u.a. eine korrekte und schadenfreie Einführung der Embolieschutzvorrichtung, beispielsweise in einen Katheter. Mit anderen Worten, die Proximal und/oder Distalform, die sich in der Grundform der Embolieschutzvorrichtung zum Inneren des Rahmens hin erstreckt, wird durch die flache oder runde Öffnung in die entgegengesetzte Richtung, also nach außen, umgeklappt.

Ein dritter Aspekt der Erfindung betrifft ein Verfahren zum Falten der erfindungsgemäßen Embolieschutzvorrichtung mittels der Formvorrichtung. Die Embolieschutzvorrichtung weist alle oder zumindest einige der genannten Merkmale auf - diese werden an dieser Stelle nicht erneut einzelnen wiedergegeben. Das Verfahren umfasst die Schritte:
Schieben des Rahmens der Embolieschutzvorrichtung vor die flache oder runde Öffnung der Formvorrichtung, wobei die Zuführungseinheit durch die Formvorrichtung hindurchgeführt wird;
Einziehen der Proximalform in die Formvorrichtung, wobei die Proximalform nach außen umgeklappt wird; Einhaken der Distalform über den äußeren Rand des distalen Teilbereichs der Formvorrichtung, wobei durch weiteres Ziehen die Distalform nach außen umgeklappt und in die Formvorrichtung eingezogen wird.

In einer Weiterentwicklung des Verfahrens ist vorteilhaft vorgesehen, dass durch das Einziehen des Rahmens in die Formvorrichtung, der Rahmen zusammengeschoben und langgestreckt wird. Durch die sich verjüngende Form des distalen Teilbereichs der Formvorrichtung wird der Rahmen von beiden Seiten zusammengeschoben, sodass er beim Austritt in den engsten Querschnitt der Formvorrichtung eine langgestreckte Form hat.

In einer Weiterentwicklung des Verfahrens ist vorteilhaft vorgesehen, dass die Embolieschutzvorrichtung aus dem vorgenannten Schlauch, der die gefaltete Embolieschutzvorrichtung enthält und der z.B. eine kommerziell erhältliche Einführschleuse sein kann, in einen im Aortenbogen vorplatzierten Katheter geschoben wird. Ein Hämostaseventil am proximalen Ende des Katheters dient hierbei zur Aufnahme und Fixierung des Schlauches und minimiert gleichzeitig den Blutverlust während der Platzierung. Durch einen Vorschub an der Zuführungseinheit wird die Embolieschutzvorrichtung nun aus dem Schlauch in den Katheter geschoben. Sobald der Rahmen vollständig im Katheter liegt, kann der Schlauch entfernt werden und über die Zuführungseinheit abgezogen werden. Die Embolieschutzvorrichtung kann dann durch Vorschub an der Zuführungseinheit über das distale Ende des Katheters hinaus in denAortenbogen geschoben werden.

In einer anderen Weiterentwicklung des Verfahrens ist vorteilhaft vorgesehen, dass die Embolieschutzvorrichtung aus dem vorgenannten Schlauch, der z.B. ein kommerziell erhältlicher Katheter sein kann, in eine im Aortenbogen vorplatzierte Schleuse geschoben wird. Durch einen Vorschub des Schlauches durch die Schleuse kann die Embolieschutzvorrichtung im Schlauch bis zum distalen Ende der Schleuse im Aortenbogen geschoben werden.

In allen Weiterbildungen der erfindungsgemäßen Embolieschutzvorrichtung ist vorgesehen, dass die umgeklappte Proximalform eine Vorspannung auf den Rahmen überträgt, die im Wesentlichen gleich ist mit der Spannung, die daraus resultiert, dass die gebogene Proximalform begradigt wird.

Des Weiteren ist auch ein Verfahren zum Entfalten der Embolieschutzvorrichtung aus einem sie enthaltenden Katheter vorgesehen. Von der Erfindung ist auch umfasst, dass die Embolieschutzvorrichtung durch beispielsweise das Verfahren zum Falten der Embolieschutzvorrichtung in eine längliche oder zylinderförmige oder Katheter-ähnliche Vorrichtung aufgenommen werden kann. Beim Entfalten der Embolieschutzvorrichtung aus dem Katheter wird diese zunächst herausgeschoben bis der distale Bereich des Rahmens den Katheter verlassen hat. Durch weiteren Vorschub der Embolieschutzvorrichtung aus dem Katheter wird die Distalform herausgeschoben, welche zurück in den Innenbereich des Rahmens umklappt. Durch das Umklappen der Distalform wird der Rahmen im distalen Bereich wieder in den Zustand der Vorspannung vor dem Falten der Embolieschutzvorrichtung gebracht. Das Umklappen der Distalform dient zum einen als Orientierungshilfe und ermöglicht eine Formänderung des Rahmens der Embolieschutzvorrichtung, so dass diese in nahezu jeden Katheter einführt werden kann. Darüber hinaus ist das Umklappen der Distalform atraumatisch.

An ihrem proximalen Bereich kann die Zuführungseinheit zwei Markierungen aufweisen, wobei die erste beim späteren Positionieren der Embolieschutzvorrichtung durch den Führungskatheter anzeigt, dass die Distalform direkt vor der Austrittsöffnung des Katheters liegt und die zweite Markierung zeigt an, dass der Rahmen den Katheter vollständig verlassen hat.

In einer weiteren vorteilhaften Weiterentwicklung wird die Richtung des Rahmens über einen oder mehrere Marker angezeigt. Die Marker können röntgenopake Marker sein. Die Marker können insbesondere im distalen Bereich des Rahmens angeordnet sein. Der distale Bereich gibt die Richtung des Rahmens beim Vorschub aus dem Katheter an. Dies hat den Vorteil, dass zum einen die genaue Position des Rahmens, seine Vorschubrichtung und die Platzierungsposition genau festgestellt werden kann.

Weitere Einzelheiten der Erfindung sind den Ausführungsbeispielen zu entnehmen, die im Folgenden anhand der Figuren beschrieben werden. Alle im Weiteren aufgeführten Einzelheiten der Erfindung sind nicht auf die angegebenen Ausführungsbeispiele beschränkt, sondern können auch einzeln, selektiv gemeinsam oder insgesamt in anderen Ausführungsbeispielen auftreten.

Es zeigen:
Figur 1: Embolieschutzvorrichtung gemäß der Erfindung;
Figur 2: Draufsicht des Rahmens der Embolieschutzvorrichtung aus Figur 1;
Figur 3: Seitenansicht des Rahmens aus Figur 2;
Figur 4: perspektivische Ansicht des proximalen Bereichs des Rahmens aus Figur 2;
Figur 5A: Rahmen mit einer Konfiguration von röntgenopaken Markern;
Figur 5B: Rahmen mit einer weiteren Konfiguration von röntgenopaken Markern;
Figur 6: Verbindung einer erfindungsgemäßen Proximalform mit der Zuführungsvorrichtung;
Figur 7: Darstellung eines Klebetunnels im Schnitt mit Rahmen, Filtereinheit und Versiegelung;
Figur 8: Draufsicht auf einen Rahmen mit daran angeordneter Filtereinheit;
Figur 9: distale Filtereinheit aus Figur 8;
Figur 10: Draufsicht auf eine Proximalform mit angeordneter Filtereinheit;
Figur 11: perspektivische Ansicht der Proximalform aus Figur 10;
Figur 12: Draufsicht auf eine Distalform mit angeordneter Filtereinheit;
Figur 13: perspektivische Ansicht der Distalform aus Figur 12;
Figur 14: Ansicht einer gefalteten Embolieschutzvorrichtung in einem Katheter;
Figur 15A-F: Umformung eines Rahmens einer erfindungsgemäßen Embolieschutzvorrichtung aus einem gefalteten Zustand in einen entfalteten Zustand;
Figur 16: schematische Ansicht eines entfalteten Zustands der Embolieschutzvorrichtung nach Verlassen eines Katheters;
Figur 17: Ablauf eines Entfaltens einer Embolieschutzvorrichtung nach Verlassen des Katheters in einem Aortenbogen;
Figur 18: Abdeckung der Kopfgefäßabgänge in der Aorta durch die Embolieschutzvorrichtung nach Verlassen des Katheters wie in Figur 17;
Figur 19: Formvorrichtung zur Umformung einer erfindungsgemäßen Embolieschutzvorrichtung;
Figur 20: perspektivische Ansicht der Formvorrichtung aus Figur 19;
Figur 21: Verfahren zum Falten der erfindungsgemäßen Embolieschutzvorrichtung mittels einer Formvorrichtung.

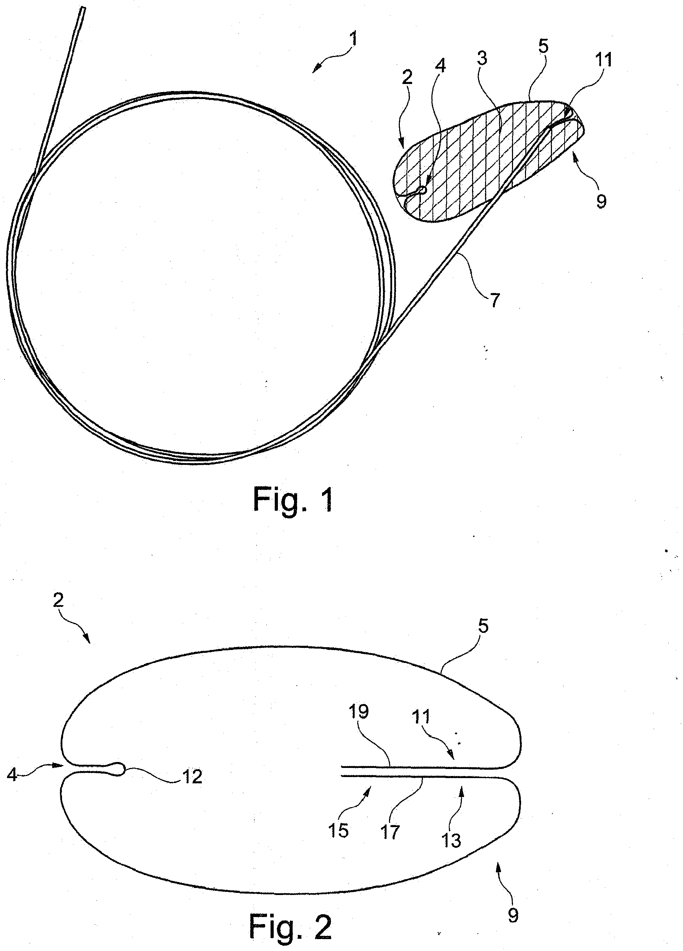

In Figur 1 ist eine Embolieschutzvorrichtung 1 gemäß der Erfindung dargestellt. Die Embolieschutzvorrichtung 1 umfasst einen Rahmen 5 an dem eine Filtereinheit 3 angeordnet ist. Der Rahmen 5 ist mit einer Zuführungseinheit 7 verbunden. Die Länge des Rahmens 5 ist vorteilhafterweise 50 bis 100 mm. Die Breite des Rahmens 5 ist vorteilhafterweise 15 bis 45 mm. In diesem Ausführungsbeispiel besteht der Rahmen 5 aus einem einzigen durchgängig gebogenen Draht. Jedoch gelten die beschriebenen Eigenschaften und Vorteile der Embolieschutzvorrichtung auch für andere Ausführungsbeispiele.

Der Rahmen 5 weist einen zweidimensionalen und einen dreidimensionalen Bereich auf. Der zweidimensionale Bereich, also die Ebene die vom Rahmen aufgespannt wird, hat eine ovale Form, die am distalen und proximalen Bereich 2, 9 in eine Proximalform 11 und Distalform 4 übergeht. Die Proximalform 11 und die Distalform 4 sind der dreidimensionale Bereich des Rahmens 5, wobei der restliche Bereich des Rahmens 5 den zweidimensionalen Bereich, also die ovale Form, bildet. Die Embolieschutzvorrichtung 1 ist in ihrer Grundform gezeigt.

Figur 2 zeigt eine Draufsicht des Rahmens 5 der Embolieschutzvorrichtung 1 aus Figur 1. Der proximale Bereich 9 des Rahmens 5 ist derjenige, der in den offenen Enden 17, 19 des Rahmens 5, in diesem Ausführungsbeispiel den Enden des Drahtes, mündet. Der proximale Bereich 9, und damit auch die Proximalform 11, ist definiert durch die beiden losen Enden 17, 19 des verwendeten Rahmens 5 bzw. Drahtes. Die Proximalform 11 weist einen ersten Teil 13 und einen zweiten Teil 15 auf, die in dieser Ausführungsform von den parallelen Enden 17, 19 ausgebildet sind. Im distalen Bereich 2 geht der Rahmen 5 in eine Distalform 4 über. Die Distalform 4 weist eine Einschnürung 12 des Drahtes in das Innere des Rahmens 5, oder anders ausgedrückt in das Innere des ovalen zweidimensionalen Bereichs, von ungefähr 1-3cm auf.

In diesem Ausführungsbeispiel ist die Einschnürung 12 eine Schlaufe mit einem Kopfdurchmesser von etwa 1-1,8 mm und dem ansonsten parallel zueinander liegenden Draht. Die Schlaufe und der parallel zueinander liegende Draht befinden sich in der gleichen zweidimensionalen Ebene des Rahmens 5.

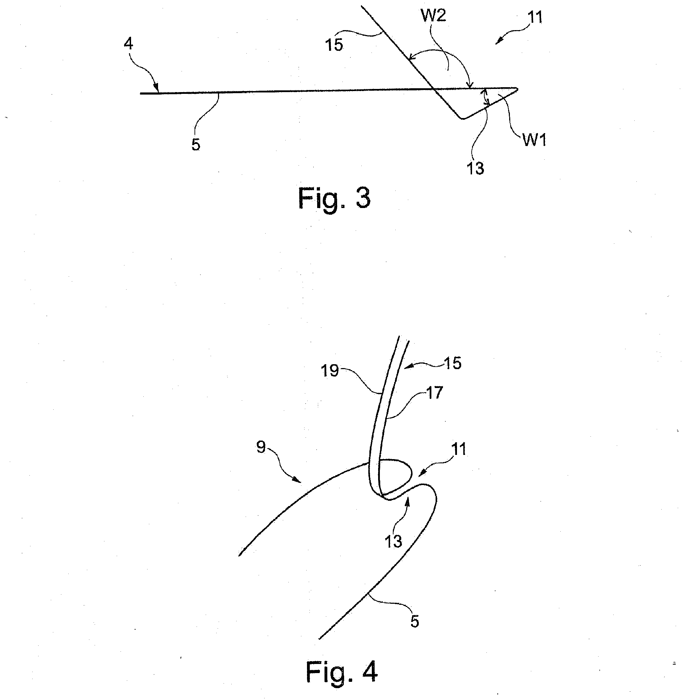

Figur 3 zeigt eine Seitenansicht des Rahmens 5 aus Figur 2 Die Proximalform 11 erstreckt sich mit den Enden des Rahmens 17, 19 wie auch der Rahmen in der Distalform 4 parallel zusammen in das Innere des Rahmens 5. Dabei weist der erste Teil 13 der Proximalform zur zweidimensionalen Ebene des Rahmens 5 einen ersten Winkel W1 von vorzugsweise 25 bis 50 Grad nach unten auf, wobei der Winkel vom ersten Teil 13 zur Ebene des Rahmens hin gemessen wird. Nach vorzugsweise 0,5 bis 2,5 cm Länge des ersten Teils 13 ist ein zweiter Teil 15 am Ende des ersten Teils 13 in einem zweiten Winkel W2 von vorzugsweise 110 bis 145 Grad nach oben aus der zweidimensionalen Ebene des Rahmens 5 angeordnet, wobei der Winkel vom zweiten Teil 15 zur Ebene des Rahmens hin gemessen wird. Die Länge des zweiten Teils 15 beträgt 1 bis 5 cm. Die Längen des ersten und zweiten Teils 13, 15 sowie auch deren Winkel zur Ebene des Rahmens 5 können auch größer oder kleiner gewählt werden entsprechend den Anforderungen an die Embolieschutzvorrichtung.

Der erste und zweite Teil 13, 15 bilden die Proximalform 11, welche in einem Innenbereich des Rahmens 5 angeordnet ist, wobei sich die Proximalform 11 oberhalb und unterhalb der Ebene des Rahmens 5 erstreckt. Durch diese geometrische Form der Proximalform 11 wird der Rahmen 5 in eine Vorspannung gebracht und zugleich in Längs- und Querrichtung stabilisiert.

Es ist möglich, dass sich der erste Teil 13 in die Ebene des Rahmens 5 erstreckt, also der Winkel W1 gleich 0 Grad ist und nur der zweite Teil 15 zur Ebene des Rahmens 5 um einen zweiten Winkel W2 geneigt ist.

Die Distalform 4, welche die Einschnürung 12 umfasst, liegt in der zweidimensionalen Ebene des Rahmens 5.

Figur 4 zeigt eine perspektivische Ansicht des proximalen Bereichs 9 des Rahmens aus Figur 2. Die Proximalform 11 umfasst den ersten Teil 13, der um einen ersten Winkel W1 zur Ebene des Rahmens 5 gebogen ist, den zweiten Teil 15 der um einen zweiten Winkel W2 zur Ebene des Rahmens 5 gebogen ist sowie die zwei Enden 17, 19 des Rahmens 5. Sowohl der erste Teil 13 als auch der zweite Teil 15 der Proximalform 11 weist jeweils zwei Rahmendrähte auf.

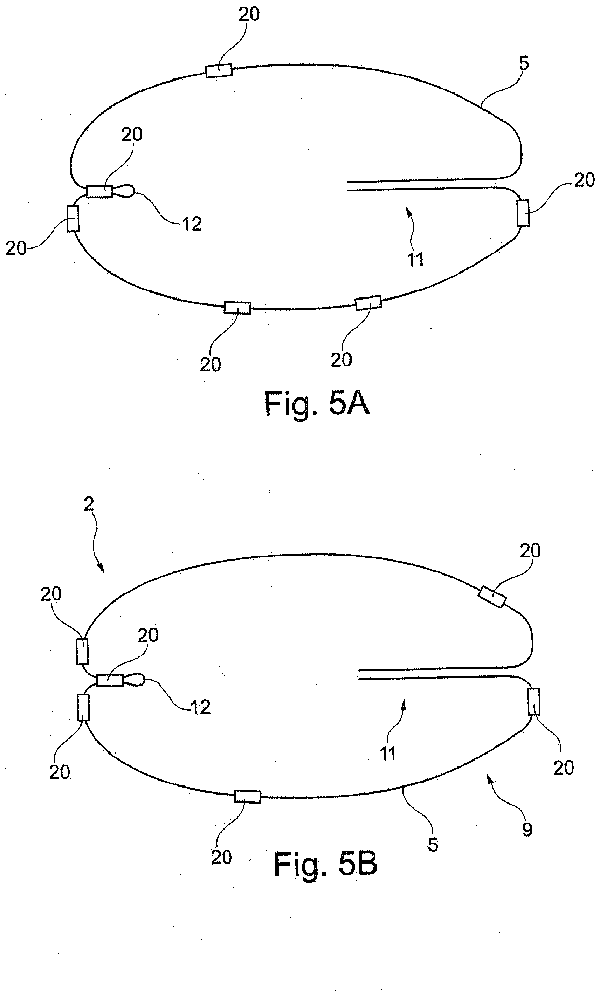

Figur 5A und 5B zeigen einen Rahmen 5 mit einer Konfiguration von röntgenopaken Markern 20. Die röntgenopaken Marker 20 sind zur röntgenopaken Sichtbarkeit auf dem Rahmen 5 an markanten Stellen, angebracht. Beispielsweise sind die röntgenopaken Marker 20 im Bereich der Einschnürung 12 sowie am Rahmen im distalen Bereich 2 angebracht, so dass die genaue Position der Spitze des Rahmens 5 ermittelt werden kann. Des Weiteren sind röntgenopake Marker 20 am Rahmen 5 außerhalb des distalen oder proximalen Bereichs 2, 9 angebracht. Durch den Abstand der Marken 20 voneinander lässt sich ermitteln in welchem Stadium das Falten oder Entfalten der Embolieschutzvorrichtung 1 ist. Auch lässt sich mittels der röntgenopaken Marker 20 die genaue Position der Embolieschutzvorrichtung 1 im Aortenbogen ermitteln.

Die röntgenopaken Marker können Hülsen aus Platin/Iridium Hülsen sein, die auf den Rahmen aufgeschoben oder aufgesteckt werden. Die Hülsen besitzen einen minimal größeren Innendurchmesser als der Rahmen 5, haben eine Wandstärke von etwa 50-100 µm und sind mittels eines Klebers fixiert.

In den Figuren 5A, 5B sind nur einige Möglichkeiten für die Positionierung von röntgenopaken Markern gezeigt. Es gibt darüber hinaus verschiedene Möglichkeiten röntgenopake Marker 20 anzubringen gemäß dem Ergebnis welches erzielt werden soll.

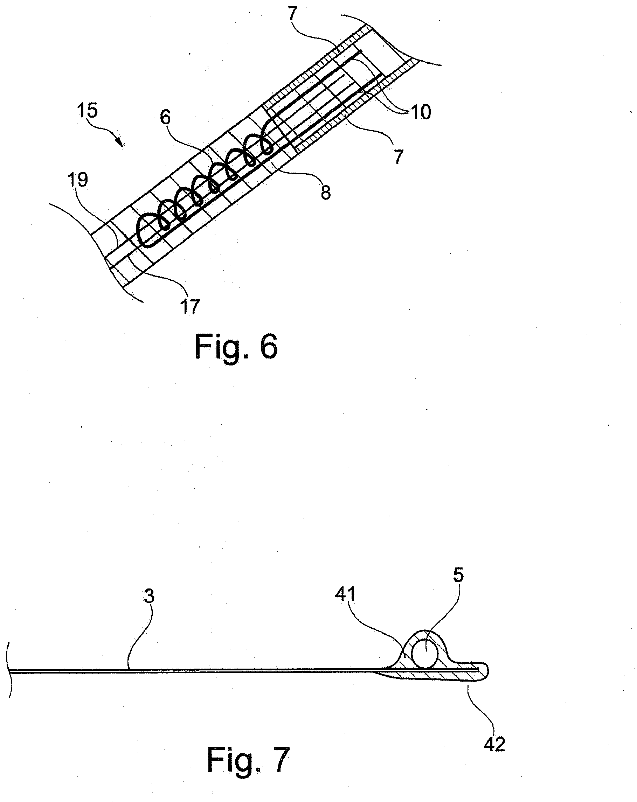

Figur 6 zeigt eine Verbindung einer erfindungsgemäßen Proximalform 11 mit einer erfindungsgemäßen Zuführungsvorrichtung 7, wobei in der Zeichnung die Enden 17, 19 des Rahmens 5 der Proximalform 11 gezeigt sind. Die Enden 17, 19 sind gleichzeitig auch das Ende des zweiten Teils 15 der Proximalform 11.

Die Zuführungseinheit 7 umfasst in diesem Ausführungsbeispiel eine Edelstahlwendel und besitzt eine Versiegelung der Ummantelung. In diesem Ausführungsbeispiel beträgt der Außendurchmesser der Zuführungseinheit 7 1,5 mm und ihr offenes Lumen hat einen Durchmesser von 0,8 mm. Die Gesamtlänge der Zuführungseinheit 7 beträgt 150 cm. Andere Maße für die Zuführungseinheit 7 sind möglich.

Die Anbindung der Proximalform 11 des Rahmens 5 an die Zuführungseinheit 7 erfolgt über eine Klebeeinheit 8, beispielsweise Polyurethankleber. Die Drahtenden 17, 19 der Proximalform 11 sind in das offene Innenlumen der Zuführungseinheit 7 eingeschoben und verklebt. Zur besseren Veranschaulichung ist die Klebeeinheit 8 in der Figur schraffiert gezeichnet.

Zur zusätzlichen Stabilisierung sind die Drahtenden 17, 19, also der zweite Teil 15 der Proximalform 11, mittels eines umgewickelten Edelstahldrahtes 6 fixiert. Dabei liegen die Drahtenden 10 des umgewickelten Edelstahldrahtes 6 parallel zu den Enden 17, 19 des Rahmens 5 verklebt in der Zuführungseinheit 7. Der Übergang von der Proximalform 11 zur Zuführungseinheit 7, sowie der umgewickelte Edelstahldraht 6 sind ebenfalls mit Polyurethan beschichtet, um eine glatte Oberfläche sowie einen gleichmäßigen Übergang zu gewährleisten.

Figur 7 zeigt eine vergrößerte Darstellung der Verbindung von Rahmen 5 und Filtereinheit 3, im Schnitt dargestellt. Die Verbindung ist als umhüllende Polymerform um den Rahmen 5 ausgeführt. Die Polymerform bildet einen Klebetunnel 41 aus, in dem der Rahmen 5 auf der Filtereinheit 3 angeordnet ist. Am äußeren Rand der Filtereinheit 3 ist in dieser Abbildung die Versiegelung 42 dargestellt.

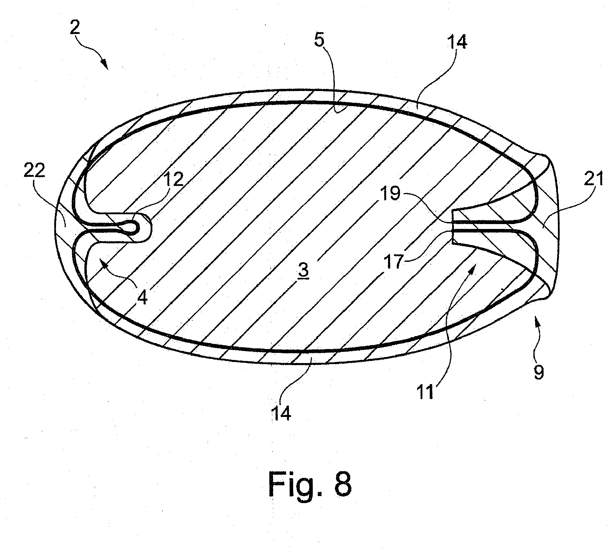

Figur 8 zeigt eine Draufsicht auf einen Rahmen 5 mit daran angeordneter Filtereinheit 3. Die Länge des Rahmens 5 ist vorteilhafterweise 50 bis 100 mm. Die Breite des Rahmens 5 ist vorteilhafterweise 15 bis 45 mm. Die Filtereinheit 3 ist in diesem Ausführungsbeispiel mittels eines Klebers oder eines Polyurethans basierten Klebers am Rahmen 5 fixiert. Die Verklebung verläuft durchgehend am äußeren Teil des Rahmens 5. Die Teile des Rahmens 5 im proximalen und distalen Bereich, die in der Grundform nach innen geklappt sind, werden nicht mit der Filtereinheit 3 verklebt. Die Filtereinheit 3 ist von der Unterseite an den Rahmen 5 geklebt, so dass die Oberfläche der Filtereinheit 3 vollständig dem zentralen Blutstrom zugewandt ist, wenn der Rahmen 5 in seiner Platzierungsposition im Aortenbogen positioniert ist.

Der Rahmen 5, in diesem Ausführungsbeispiel aus Nitinol, wird in einer Vorspannung mit der Filtereinheit 3 verklebt, um eine verbesserte Aufspannkraft des Rahmens 5 zu erreichen. Dabei wird die Breite des Rahmens 5 von 35-45mm auf 25-35mm gestaucht.

Die Filtereinheit 3 ragt mit einen Überstand 14 von etwa 1 mm über den Rahmen 5 auf dessen Oberseite und ist im distalen und proximalen Bereich 2, 9 des Rahmens 5 von der Unterseite über den Rahmen 5 zur Oberseite umgelegt oder umgeklappt. Der Überstand 14 der angebrachten Filtereinheit 3 über die Außenkante des Rahmens 5 hinweg hat die zusätzliche Funktion einer flexiblen Dichtungslippe zur Aortenwand hin, wenn die Embolieschutzvorrichtung 1 in der Platzierungsposition im Aortenbogen liegt.

Der umgelegte Bereich der Filtereinheit 3 umfasst eine proximale Filtereinheit 21 und eine distale Filtereinheit 22. Die proximale und distale Filtereinheit 21, 22 sind nicht mit Kleber am Rahmen fixiert, so dass eine gewünschte Verformung beim vorgesehenen Durchschub durch einen Katheter begünstigt ist. Die proximale Filtereinheit 21 ist zusammen mit dem zweiten Teil 15 der Proximalform 11 unter dem umgewickelten Edelstahldraht 6 fixiert und in diesem Bereich versiegelt, siehe auch Darstellung in Figur 7.

Die distale Filtereinheit 22 wird an der Einschnürung 12 der Distalform 4 befestigt. Die distale Filtereinheit 22 erstreckt sich über die Einschnürung 12 weiter in das Innere des Rahmens 5 hinein, etwa 2-5 mm und ist zusätzlich flexibel versiegelt.

Die Fasern der Filtereinheit 3 sind so ausgerichtet, dass sie in einem 45° Winkel zur Mittellinie des Rahmens 5 von Anfang bis Ende ausgerichtet sind. Dies erlaubt der Filtereinheit 3 sich besser in Längsrichtung zu dehnen, während die Querrichtung Stabilität bietet. Der äußere Rand der Überstande 14 sowie 21 und 22 sind zusätzlich versiegelt.

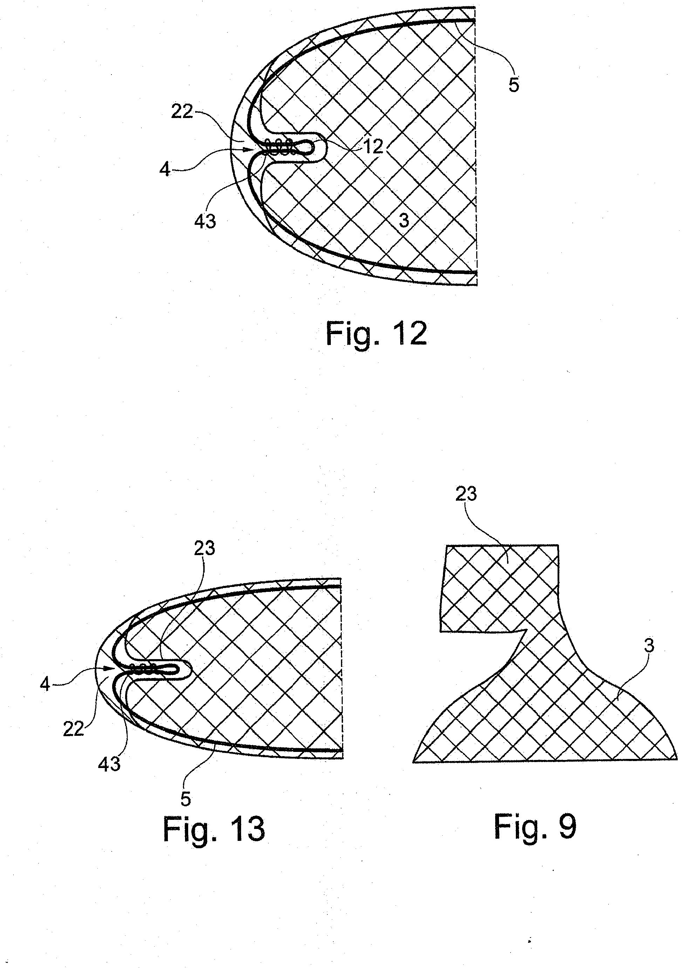

Figur 9 zeigt einen Endbereich der distalen Filtereinheit 22 der Filtereinheit 3 aus Figur 8. Die distale Filtereinheit 22 ist so zugeschnitten, dass sie sich nicht nur ca. 2 mm über die Einschnürung 12 erstreckt, sondern hinter der Einschnürung 12 wieder breiter wird und die Form eines Fähnchens 23 annimmt.

Dieses Fähnchen 23 wird eingerollt. Dabei werden die Enden des verwendeten Fadens zur Fixierung im Inneren des Fähnchens 23 eingeschlossen. Kleber fixiert die distale Filtereinheit 23 gegen ein Aufrollen. Der Durchmesser der eingerollten distalen Filtereinheit 22 beträgt weniger als 1,6 mm. Außer einer Fixierung der distalen Filtereinheit 23 wird somit auch ein zusätzliches Schutzpolster zwischen Rahmen 5 und Aortenwand gebildet, um Verletzungen vorzubeugen.

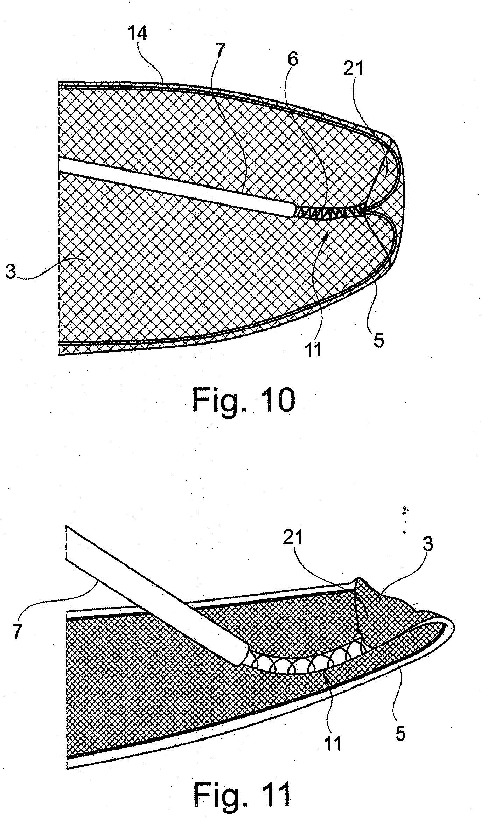

Figur 10 zeigt eine Draufsicht auf eine Proximalform 11 mit angeordneter Filtereinheit 3. Die proximale Filtereinheit 21 ist um den Rahmen 5 zur Oberseite hin umgelegt. In diesem Ausführungsbeispiel sind sowohl der erste Teil 13 der Proximalform 11 als auch der zweite Teil 15 der Proximalform 11 mit einem Edelstahldraht 6 umwickelt (zur besseren Veranschaulichung des Edelstahldrahtes ist der erste und zweite Teil 13, 15 der Proximalform nicht gezeigt). Der erste Teil 13 weist einen ersten Winkel W1 zur Ebene des Rahmens 5 auf und der zweite Teil 15 ist um einen zweiten Winkel W2 zur der Ebene des Rahmens 5 gebogen.

Figur 11 zeigt perspektivische Ansicht der Proximalform 11 aus Figur 10.

Figur 12 zeigt eine Draufsicht auf eine Distalform 4 mit angeordneter Filtereinheit 3, insbesondere eine distale Filtereinheit 22. Die distale Filtereinheit 22 wird mittels eines Fadens 43, welcher in anderen Ausführungsformen auch ein Garn oder Draht sein kann, an der Einschnürung 12 der Distalform 4 befestigt und ragt in das Innere des Rahmens 5.

Figur 13 zeigt eine Draufsicht auf eine Proximalform 11 mit angeordneter Filtereinheit 3. Das Fähnchen 23 ist eingerollt, wobei zu seiner Fixierung ein Faden verwendet wird, wie bei Figur 9 beschrieben und hier nicht wiederholt wird.

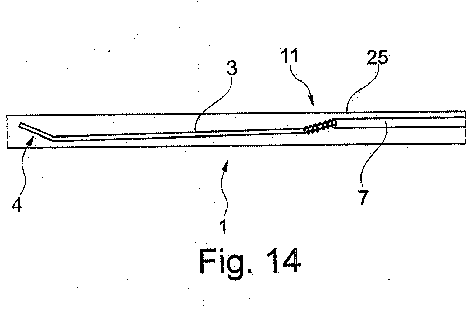

Figur 14 zeigt eine gefaltete Embolieschutzvorrichtung 1 in einem Katheter 25. Die Embolieschutzvorrichtung 1 ist in einem gefalteten Zustand. Durch mechanische Umformung wird die Grundform der Embolieschutzvorrichtung, wie beispielsweise in den Figuren 1 bis 13 gezeigt, in den gefalteten Zustand überführt. Das reversibel verformbare Material des Rahmens 5, beispielsweise ein superelastischer Nitinoldraht, lässt sich so verformen, dass sich die Embolieschutzvorrichtung 1 in einen Katheter 25 schieben lässt. Die Embolieschutzvorrichtung 1 streckt sich dabei längs ihrer Richtung. Die Distalform 4 als auch die Proximalform 11 sind in einen Außenbereich des Rahmens 5 umgeklappt.

Durch das Umklappen der Distalform 4 und der Proximalform 11 geht der Rahmen 5 in eine gerade oder gestreckte Form über. Die damit einhergehende Längenänderung ist bedingt durch die Verringerung der Breite des Rahmens 5. Der gefaltete Rahmen 5, d.h. die zwei Seiten des Rahmens außerhalb der Distalform 4 und/oder Proximalform 11, liegen dabei parallel zueinander im Katheter 25. Die Filtereinheit 3 kann dieser mechanischen Verformung folgen und legt sich in den Zwischenraum zwischen Katheter 25 und Rahmen 5. In dieser gestreckten Form lässt sich die Embolieschutzvorrichtung in einen Katheter mit beispielsweise 1,7 mm Innendurchmesser schieben.

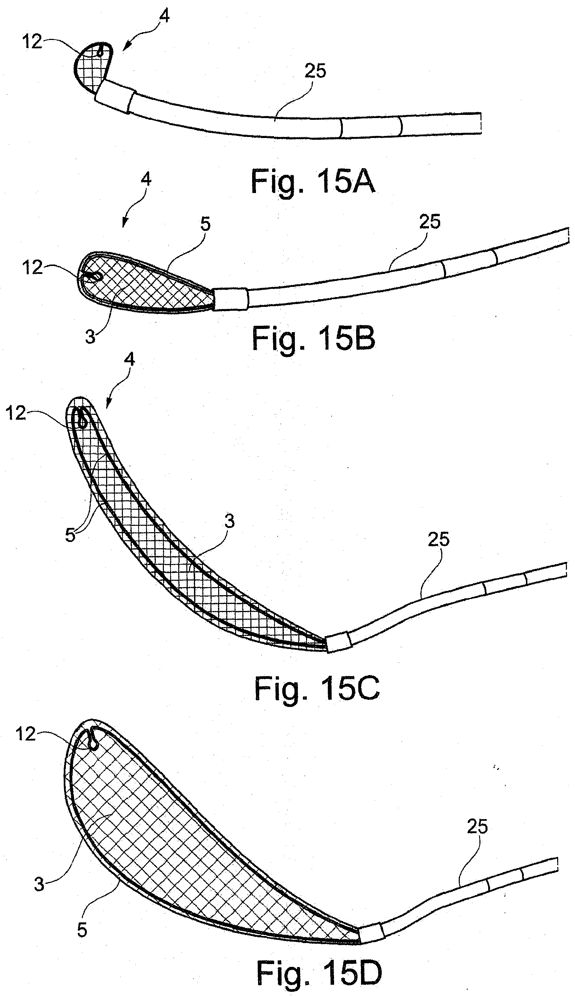

Figur 15A-F zeigt eine Umformung eines Rahmens 5 einer erfindungsgemäßen Embolieschutzvorrichtung 1 aus einem gefalteten Zustand in einen entfalteten Zustand.

Beim Positionieren der Embolieschutzvorrichtung aus einem Katheter 25, beispielsweise in den Aortenbogen, wird die Embolieschutzvorrichtung 1, insbesondere der Rahmen 5 mit der an ihm angeordneten Filtereinheit 3 aus dem Katheter 25 geschoben. Dies ist in den Figuren 15A-F gezeigt.

Der Rahmen 5, der aus reversibel verformbaren Material gebildet ist, versucht seine ursprüngliche Grundform, wie beispielsweise in Figur 1 gezeigt, wieder einzunehmen. Die am Rahmen 5 angeordnete Filtereinheit 3 folgt der Umformung. Durch den Vorschub der nach vorn geklappten im Katheter 25 liegenden Distalform 4, klappt diese beim Verlassens des Katheters 25, etwa nach ca. 1-2 cm Vorschub, zur Hälfte zurück in Richtung der ursprünglich vorgesehenen Stellung, vgl. Figur 15A. Dabei gibt die Richtung der Distalform 4 an, in welcher Position sich die Embolieschutzvorrichtung 1 im Inneren des Katheters 25 befindet. Röntgenopake Marker, die an der Distalform 4 angebracht werden können, erlauben die Erkennung der Position der Distalform 4. Die Zeigerichtung der Distalform 4 gibt dabei die Oberseite der Embolieschutzvorrichtung 1 an. Durch Drehen des Katheters 25 lässt sich die Platzierungsposition, beispielsweise im Aortenbogen, anpassen.

Da die Einschnürung 12 im distalen Bereich der Embolieschutzvorrichtung 1, also die Distalform 4 unmittelbar nach dem Verlassen des Katheters 25 umklappt, vergleiche Figur 15A-B, wird die Gefahr einer möglichen Beschädigung der Gefäßwand durch weiteren Vorschub der Embolieschutzvorrichtung 1 minimiert. Zusätzlich ist der Rahmen 5 an der Distalform 4 mit atraumatischem Material umwickelt welches einen Überstand von ca. 1-2 mm aufweist und dadurch ebenfalls möglichen Verletzungen entgegenwirkt.

Im weiteren Verlauf des Vorschubs in den Aortenbogen faltet sich der Rahmen 5 weiter auf bis er vollständig aufgeklappt ist. Dies ist beispielsweise in den Figuren 15C-F sichtbar. Figur 15D zeigt dabei denselben Entfaltungszustand wie Figur 15C aus einer seitlichen Blickrichtung, wobei Figur 15C den Entfaltungszustand aus einer Blickrichtung von oben zeigt. Die nun wieder nahezu vollständig eingeklappte Distalform 4 und der sich entfaltende Rahmen 5, sowie die sich entfaltende Filtereinheit 3 sind gezeigt.

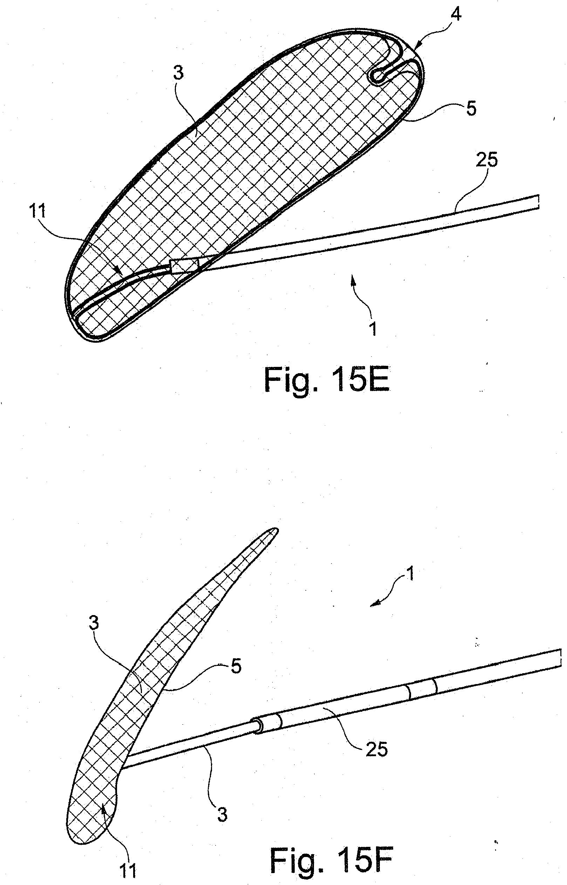

Im vollständig entfalteten Zustand ist der Rahmen 5 entfaltet und die Filtereinheit 3 wird vom Rahmen 5 aufgespannt. Figur 15E zeigt den vollständig entfalteten Rahmen bzw. die entfaltete Embolieschutzvorrichtung 1 aus einer Blickrichtung von oben und in Figur 15F aus einer seitlichen Blickrichtung. Die Wirkung des Federmechanismus durch die Proximalform 11 ist aus dem Übergang von Figur 15C zu Figur 15E oder Figur 15D zu Figur 15F ersichtlich.

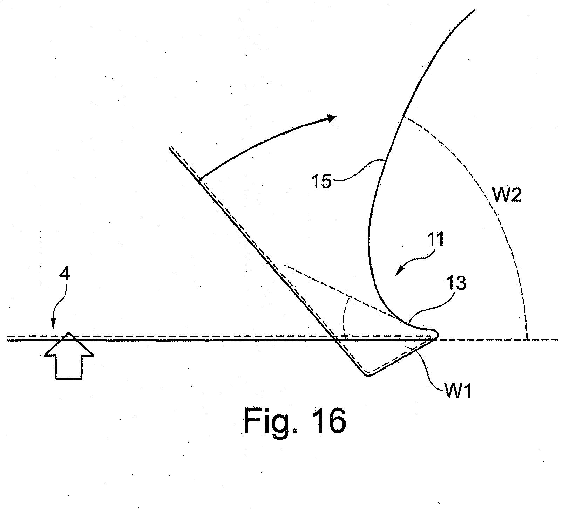

Figur 16 zeigt schematisch einen entfalteten Zustand der Embolieschutzvorrichtung 1 nach Verlassen eines Katheters 25. Durch die besondere Geometrie der Proximalform 11 bis zum Übergang zur Zuführungseinheit 3, ergibt sich eine Vorspannung auf den Rahmen 5 im gleichen Maß wie die vorgebogene Proximalform 11 begradigt wird. Die Abbildung zeigt zwei verschiedene Zustände des entfalteten Zustands. Die Position der Filtereinheit ist in beiden Darstellungen identisch. Die Lage des ersten und zweiten Teils 13, 15, welche mit der Zuführungseinheit (nicht gezeigt) verbunden sind, ist sowohl im entspannten, wie auch im gespannten Zustand dargestellt. Daraus ergibt sich eine Federfunktion, die im Weiteren genauer erläutert wird.

Sobald die Embolieschutzvorrichtung 1 korrekt, beispielsweise im Aortenbogen, positioniert ist, drückt die übertragene Spannung der Proximalform 11 die Distalform 4 an die Aortenwand und erlaubt so eine stabile Fixierung gegen den Blutstrom - in Figur 16 ist dies durch den dicken kurzen Pfeil an der Distalform 4 skizzenhaft angezeigt. Die Proximalform 11 folgt einer Bewegung, die mittels des dünnen gebogenen Pfeils dargestellt ist. Ohne den Wiederstand der Aortenwand würde der Rahmen 5 der eingezeichneten Klapprichtung folgen - in Figur 16 der gebogene dünne Pfeil - wie beispielsweise in Figur 15E-F gezeigt. Wie in Figur 16 gezeigt, geht die Proximalform 11 in eine Form über, in der der erste Teil 13 einen ersten Winkel W1 von 25 bis 50 Grad oberhalb der Ebene des Rahmens aufweist, gemessen von der Ebene zum ersten Teil 13 hin und der zweite Teil 15 einen Winkel W2 von 30 bis 110 Grad oberhalb der Ebene des Rahmens aufweist, gemessen vom zweiten Teil 15 zur Ebene hin. Angegebenen Gradzahlen der Winkel sind abhängig von der Aortengeometrie und sind nur beispielhaft.

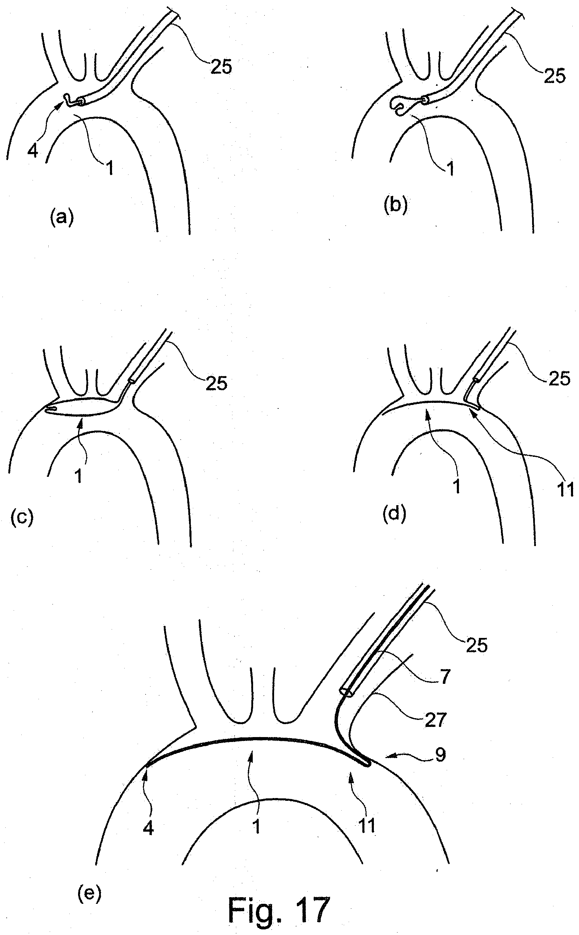

Figur 17 zeigt einen schematischen Ablauf eines Entfaltens einer Embolieschutzvorrichtung 1 nach Verlassen eines Katheters 25 in einem Aortenbogen. Im Figurteil (a) ist die Einbringung des Katheters 25 durch die linke Arteria Subclavia gezeigt, wobei die Distalform 4 der Embolieschutzvorrichtung 1 zumindest teilweise zurückgeklappt ist. Figurteile (b) bis (d) zeigen den weiteren Vorschub und das Entfalten der Embolieschutzvorrichtung 1, wobei im Figurteil (d) auch die Proximalform 11 den Katheter verlassen hat. Figurteil (e) zeigt die vollständig entfaltete Embolieschutzvorrichtung 1 im Platzierungszustand. Der proximale Bereich 9 des Rahmens 5 ragt dabei über die Fläche das Ostiums der linken Arteria Subclavia hinaus, wodurch eine Abdeckung mittels der Embolieschutzvorrichtung 1 auch über den Zugangsweg hinweg erreicht wird. Dieser Überstand bietet gleichzeitig eine haptische Rückmeldung beim Positionieren der Embolieschutzvorrichtung: durch Ziehen an der Zuführungseinheit 7 ist ein leichter Widerstand fühlbar sobald der Überstand der Embolieschutzvorrichtung 1, genauer gesagt des Rahmens 5, korrekt vor dem Ostium 27 liegt. Die vorgesehene Position erfolgt über die linke Arteria Subclavia in den Aortenbogen mit dem distalen Bereich 2 des Rahmens 5 Richtung Herzklappe.

Als alternativer Zugangsweg kann auch die rechte Arteria Subclavia dienen. Der Ablauf ist ähnlich wie in Figur 16 gezeigt, jedoch in gespiegelter Ausführung. Hierbei zeigt der distale Bereich 2 des Rahmens 5 in Richtung absteigende Aorta (Descendens).

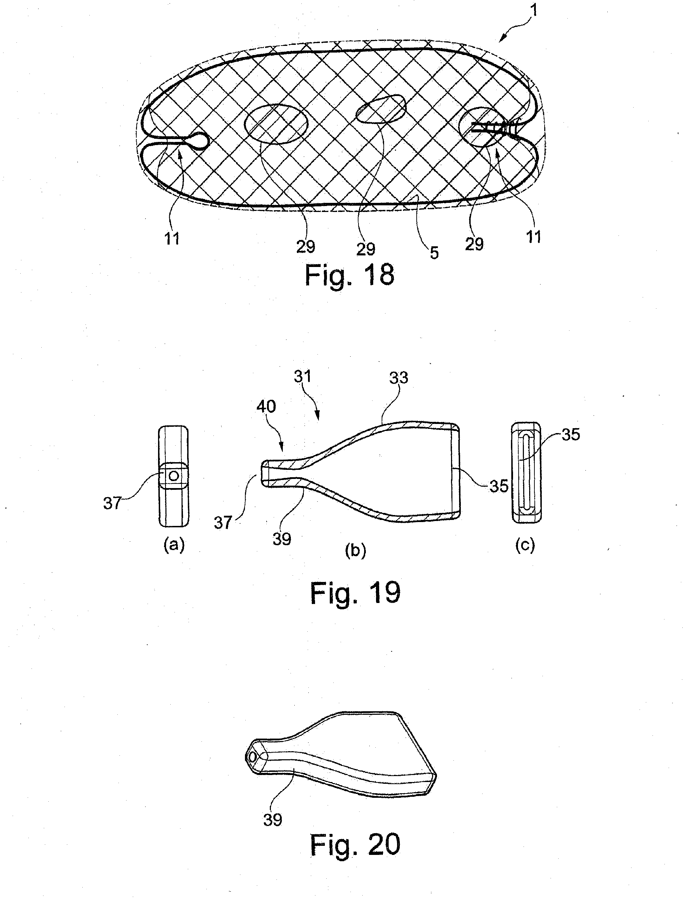

Figur 18 zeigt eine Abdeckung der Kopfgefäßabgänge 29 in der Aorta durch die Embolieschutzvorrichtung 1 nach Verlassen des Katheters 25 wie in Figur 17. Durch die besondere Geometrie des Rahmens 5 passt sich die Embolieschutzvorrichtung 1 den anatomischen Bedingungen im Aortenbogen flexibel und unabhängig von dem Zugangsweg an und bietet eine komplette Abdeckung über alle Kopfgefäße 29.

In der Platzierungsposition im Aortenbogen passt sich die Geometrie des Rahmens 5 der Embolieschutzvorrichtung 1 flexibel der Aortenwand an und liegt in einem leichten Bogen, der Krümmung der Aorta folgend, vor den Kopfgefäßabgängen - vgl. auch Figur 17(e). Beim Verlassen des Katheters 25 klappt sowohl die Distalform 4 als auch die Proximalform 11 in Richtung ihrer ursprünglichen Form zurück, also in Richtung des Innenbereichs des Rahmens 5 und ermöglichen so eine atraumatische Positionierung des Rahmens 5 an der Aortenwand. Durch das Umklappen werden scharfkantige Übergänge oder Ecken vermieden. Eine zusätzliche Stabilisierung des Rahmens 5 wird durch die physiologischen Bedingungen in der Aorta erreicht, da der Blutfluss den Rahmen 5 der Embolieschutzvorrichtung 1 zusätzlich in seine Platzierungsposition drückt.

Figur 19 zeigt eine Formvorrichtung 31 zur Umformung einer erfindungsgemäßen Embolieschutzvorrichtung 1 in verschiedenen Ansichten. Um die Umformung der Embolieschutzvorrichtung aus einem entfalteten Zustand in der Grundform in einen gestreckten Zustand zu vereinfachen, wird die Embolieschutzvorrichtung in den distalen Teilbereich 33 der Formvorrichtung 31 eingezogen. Der distale Teilbereich 33 der Formvorrichtung 31 weist einen flachen Trichter mit einer flachen Öffnung 35 von ca. 25-40 mm Öffnungsbreite und einer Öffnungshöhe von ca. 3-10 mm auf. Über die Länge des distalen Teilbereichs 33 der Formvorrichtung 31 von ca. 60-80 mm verjüngt sich die Öffnungsfläche der Vorderseite zu einem kreisrunden engsten Querschnitt 39, mit ca. 1,7 mm Durchmesser. Der proximale Teilbereich 40 der Formvorrichtung 31 erweitert sich vom engsten Querschnitt 39 hin zur runden Öffnung 37 auf ca. 1,8 bis 5 mm Durchmesser über eine Länge von 20 bis 40 mm. Dadurch ergibt sich eine Gesamtlänge der Formvorrichtung 31 von 80 bis 120 mm.

Figur 20 zeigt eine perspektivische Ansicht der Formvorrichtung 31 aus Figur 19.

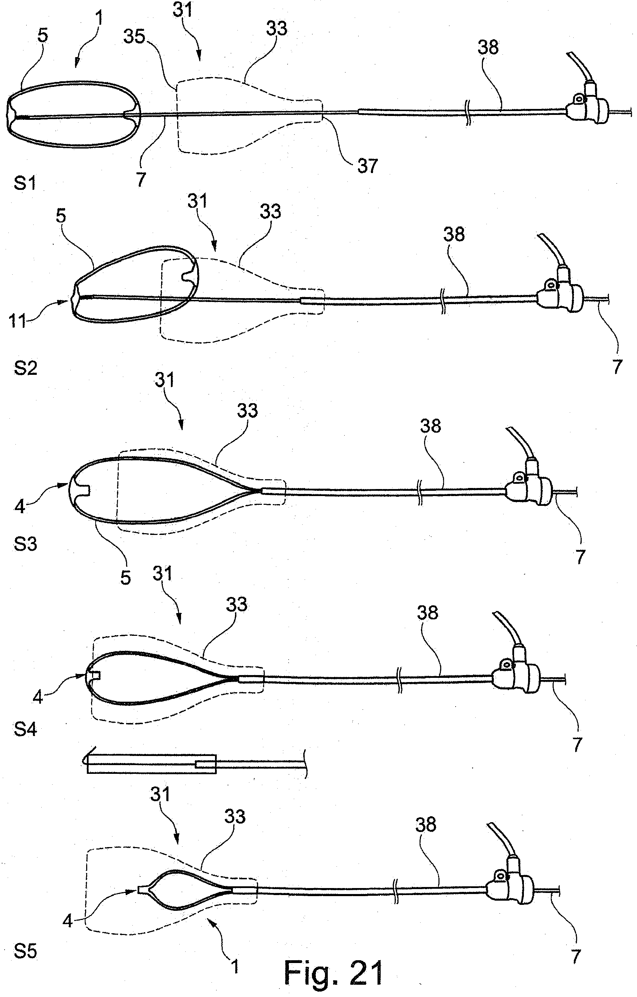

Figur 21 zeigt einzelne Schritte des Verfahrens zum Falten der erfindungsgemäßen Embolieschutzvorrichtung mittels einer Formvorrichtung 31, wobei die gefaltete Embolieschutzvorrichtung 1 in einen im Wesentlichen kreisrunden Schlauch 38, z.B. eine kommerziell erhältliche Einführschleuse oder einen kommerziell erhältlichen Katheter (mit Innendurchmesser 1,8 bis 2,5 mm) 38 geschoben wird. In einem Schritt S1 wird der Rahmen 5 der Embolieschutzvorrichtung 1, mit der Zuführungseinheit 7 voraus, vor die flache Öffnung 35 der Formvorrichtung 31 geschoben. Das proximale Ende der Zuführungseinheit 7 wird dabei durch das distale Ende der Formvorrichtung 31 hindurchgeführt. Auf die Zuführungseinheit 7 wird von proximal der im Wesentlichen kreisrunde Schlauch 38 mit seinem distalen Ende aufgeschoben, bis vor die runde Öffnung 37 der Formvorrichtung 31, wobei die Zuführungseinheit 7 aus diesem Schlauch 38 herausragt.

In einem Schritt S2 werden der Schlauch 38 und die Formvorrichtung 31 miteinander in der erweiterten oder konusförmigen runden Öffnung 37 der Formvorrichtung 31 verbunden, beispielsweise durch eine Steckverbindung. Durch Ziehen an der Zuführungseinheit 7 wird die Embolieschutzvorrichtung 1 ausgerichtet.

Durch weiteres Ziehen an der Zuführungseinheit 7 wird in einem Schritt S3 die Proximalform 11 am äußeren Rand des distalen Teilbereichs 33 der Formvorrichtung 31 umgeklappt, sodass sie gestreckt durch die Formvorrichtung 31 gezogen wird.

Weiteres Ziehen an der Zuführungseinheit 7 führt in einem Schritt S4 dazu, dass die Distalform 4 über den äußeren Rand des distalen Teilbereichs 33 der Formvorrichtung 31 geschoben wird, wobei die Distalform 4 am Rand einhakt und nach außen umgeklappt wird. Dies wird in der Abbildung ebenfalls in der Seitenansicht verdeutlicht.

In einem Schritt S5 wird die Embolieschutzvorrichtung 1 vollständig gestreckt durch die Formvorrichtung 31 gezogen. Durch weiteres Ziehen an der Zuführungseinheit 7 werden die Seiten des Rahmens 5 nach innen geschoben bis der ganze Rahmen langgestreckt in den Schlauch 38 gezogen ist. Die Embolieschutzvorrichtung 1 verbleibt in diesem Schlauch 38. Die Formvorrichtung 31 kann nun von dem Schlauch 38 entfernt werden.

Zusammenfassend ist festzuhalten, dass durch die beschriebenen Maßnahmen eine erfindungsgemäße Embolieschutzvorrichtung (1) zur Zuführung in einen Aortenbogen ausgebildet ist, umfassend eine Filtereinheit (3), einen Rahmen (5) und eine Zuführungseinheit (7), wobei die Filtereinheit (3) am Rahmen (5) angeordnet ist und der Rahmen (5) einen proximalen Bereich (9) aufweist, der eine Proximalform (11) umfasst, welche in einem Innenbereich des Rahmens (5) angeordnet ist und mit der Zuführungseinheit (7) verbunden ist, wobei die Proximalform (11) einen ersten Teil (13) und einen zweiten Teil (15) umfasst, wobei der zweite Teil (15) an einem Ende des ersten Teils (13) ausgebildet ist.

In einer Weiterbildung der Embolieschutzvorrichtung (1) weist der erste Teil (13) der Proximalform (11) zur Ebene des Rahmens (5) einen ersten Winkel (W1) auf und der zweite Teil (15) zum ersten Teil (13) der Proximalform (11) einen zweiten Winkel (W2) auf.

Erfindungsgemäß wird eine Embolieschutzvorrichtung (1) zur Zuführung in einen Aortenbogen angegeben, umfassend eine Filtereinheit (3), einen Rahmen (5) und eine Zuführungseinheit (7), wobei die Filtereinheit (3) am Rahmen (5) angeordnet ist und der Rahmen (5) einen proximalen Bereich (9) aufweist, der eine Proximalform (11) umfasst, welche in einem Innenbereich des Rahmens (5) angeordnet ist und mit der Zuführungseinheit (7) verbunden ist, wobei die Proximalform (11) einen ersten Teil (13) und einen zweiten Teil (15) umfasst, wobei der erste und zweite Teil (13, 15) derart zueinander angeordnet sind, dass sie einen Federmechanismus ausbilden.

In einer Weiterbildung der Embolieschutzvorrichtung (1) kann die Proximalform (11) über die Zuführungseinheit (7) unter Spannung gesetzt werden.

In einer Weiterbildung der Embolieschutzvorrichtung (1) umfasst die Proximalform (11) zwei Enden (17, 19) des Rahmens (5), die sich parallel zueinander im Innenbereich des Rahmens (5) erstrecken.

In einer Weiterbildung der Embolieschutzvorrichtung (1) ist die Proximalform (11) mit der Zuführungseinheit (7) verbunden, wobei die zwei Enden (17,19) des Rahmens (5) von einem Draht (6) umwickelt sind, dessen Enden (10) parallel zu den Enden (17, 19) des Rahmens (5) angeordnet sind.

In einer Weiterbildung der Embolieschutzvorrichtung (1) weist der Rahmen (5) einen distalen Bereich (2) auf, der eine Distalform (4) umfasst, welche in einem Innenbereich des Rahmens (5) angeordnet ist.

In einer Weiterbildung der Embolieschutzvorrichtung (1) weist die Distalform (4) eine zum Inneren des Rahmens (5) hin gerichtete Einschnürung (12) auf.

Erfindungsgemäß ist die Verbindung von Rahmen (5) und Filtereinheit (3) der Embolievorrichtung mittels eines Klebetunnels oder einer Klebetunnelverbindung realisiert.

In einer Weiterbildung der Embolieschutzvorrichtung (1) ist die Filtereinheit (3) außerhalb des proximalen und/oder distalen Bereichs (9, 2) mit dem Rahmen (5) verbunden.

In einer Weiterbildung der Embolieschutzvorrichtung (1) ist die Filtereinheit (3) im distalen Bereich (2) im Wesentlichen bis zum Anfang der Distalform (4) mit dem Rahmen (5) verbunden.

In einer Weiterbildung der Embolieschutzvorrichtung (1) ist die Filtereinheit (3) im proximalen Bereich (2) im Wesentlichen bis zum ersten Teil (13) der Proximalform (11) mit dem Rahmen (5) verbunden.

In einer Weiterbildung der Embolieschutzvorrichtung (1) ist die Filtereinheit (3) im distalen und proximalen Bereich (2, 9) flexibel mit dem Rahmen (5) verbunden.

In einer Weiterbildung der Embolieschutzvorrichtung (1) wird der Rahmen (5) in einer Vorspannung in Querrichtung mit der Filtereinheit (3) verbunden.

In einer Weiterbildung der Embolieschutzvorrichtung (1) weist die Filtereinheit (3) einen Überstand (14) über den Rahmen (5) auf.

In einer Weiterbildung der Embolieschutzvorrichtung (1) ist der Überstand (14) versiegelt.

In einer Weiterbildung der Embolieschutzvorrichtung (1) ist der Überstand (14) als Dichtungslippe ausgebildet.

In einer Weiterbildung der Embolieschutzvorrichtung (1) ist im proximalen und/oder distalen Bereich (9, 2) des Rahmens (5) die Filtereinheit (3) über den Rahmen (5) von der Unterseite zur Oberseite hin umgelegt.

In einer Weiterbildung der Embolieschutzvorrichtung (1) ist die Filtereinheit (3) mittels eines Fadens, Drahtes oder Garns an der Distalform (4) befestigt.

In einer Weiterbildung der Embolieschutzvorrichtung (1) ist die Befestigung der Filtereinheit (3) mittels eines Fadens, Drahtes oder Garns an der Distalform (4) versiegelt.

In einer Weiterbildung der Embolieschutzvorrichtung (1) ist die Filtereinheit (3) mittels einer Verklebung an der Distalform (4) befestigt.

In einer Weiterbildung der Embolieschutzvorrichtung (1) ist die Filtereinheit (3) mittels einer Wendel im proximalen Bereich (9) befestigt.

In einer Weiterbildung der Embolieschutzvorrichtung (1) weist die Filtereinheit (3) ein Fasermaterial auf, wobei die Fasern so ausgerichtet sind, dass sie einen Winkel von im Wesentlichen 45 Grad zu einer Längsachse des Rahmens (5) aufweisen.

In einer Weiterbildung der Embolieschutzvorrichtung (1) ist der Rahmen (5) eine Grundform aufweist, welche als ovale Form ausgebildet.

Erfindungsgemäß wird eine Formvorrichtung (31) zur Umformung der Embolieschutzvorrichtung (1) angegeben, zum Einziehen in einen Schlauch, wobei ein Rahmen (5) mit einer daran angeordneten Filtereinheit (3) der Embolieschutzvorrichtung (1) von einem expandierten Zustand in einen gestreckten Zustand umgeformt wird, umfassend eine einseitig flache oder runde Öffnung (35), einen engsten Querschnitt (39) und eine gegenüberliegende runde Öffnung (37).

In einer Weiterbildung der Formvorrichtung ist die flache oder runde Öffnung (35) der Formvorrichtung (31) so ausgebildet, dass die Proximalform (11) und/oder die Distalform (4) des Rahmens (5) der Embolieschutzvorrichtung nach außen geklappt wird.

Erfindungsgemäß wird ein Verfahren zum Falten der Embolieschutzvorrichtung mittels der Formvorrichtung angegeben, umfassend Schieben (S1) des Rahmens (5) der Embolieschutzvorrichtung vor die flache oder runde Öffnung (35) der Formvorrichtung (31), wobei die Zuführungseinheit (7) durch die Formvorrichtung (31) hindurchgeführt wird, Einziehen (S3) der Proximalform (11) in die Formvorrichtung (31), wobei die Proximalform (11) nach außen umgeklappt wird, Einhaken (S4) der Distalform (4) über den äußeren Rand der Formvorrichtung (31), wobei durch weiteres Ziehen die Distalform (4) nach außen umgeklappt und in die Formvorrichtung (31) eingezogen wird.

In einer Weiterbildung des Verfahrens wird durch das Einziehen des Rahmens (5) in die Formvorrichtung (31), der Rahmen (5) langgestreckt.

In einer Weiterbildung des Verfahrens, wobei die umgeklappte Proximalform (11) eine Vorspannung auf den Rahmen (5) überträgt, die im Wesentlichen gleich ist mit der Spannung, die daraus resultiert, wird die gebogene Proximalform (11) begradigt.

In der Beschreibung wird ein Verfahren zum Entfalten der Embolieschutzvorrichtung angegeben, wenn die Embolieschutzvorrichtung ein sie enthaltendes Katheter verlässt, umfassend Herausschieben der Embolieschutzvorrichtung aus dem Katheter,
Umklappen der Distalform (4) zurück in einen Innenbereich des Rahmens (5), wenn ein distaler Bereich (2) des Rahmens der Embolieschutzvorrichtung den Katheter verlässt.

Eine Weiterbildung des Verfahrens umfasst Anzeigen der Richtung des Rahmens (5) über einen oder mehrere Marker, wenn der distale Bereich (2) den Katheter verlässt, wobei der distale Bereich (2) die Richtung des Rahmens (5) angibt.

Eine Weiterbildung des Verfahrens wobei durch die Vorbiegung der Distalform als auch der Proximalform eine Torsion im Draht des Rahmens (5) erzeugt wird, die eine Vorzugsrichtung beim Verlassen des Katheters in Richtung der gebogenen Spitze der Distalform hat.

### Bezugszeichenliste

- 1: Embolieschutzvorrichtung
- 2: distaler Bereich
- 3: Filtereinheit
- 4: Distalform
- 5: Rahmen
- 6: Edelstahldraht
- 7: Zuführungseinheit
- 8: Klebeeinheit
- 9: proximaler Bereich
- 10: Drahtenden
- 11: Proximalform
- 12: Einschnürung
- 13: erster Teil
- 14: Überstand
- 15: zweiter Teil
- 17, 19: Enden des Rahmens
- 20: Marker
- 21: proximale Filtereinheit
- 22: distale Filtereinheit
- 23: Fähnchen
- 25: Katheter
- 27: Ostium
- 29: Kopfgefäßabgänge
- 31: Formvorrichtung
- 33: distaler Teilbereich
- 35: flache Öffnung
- 37: runde Öffnung
- 38: Schlauch
- 39: engster Querschnitt
- 40: proximaler Teilbereich
- 41: Klebetunnel
- 42: Versiegelung
- 43: Faden
- S1-S5: Verfahrensschritte
- W1: erster Winkel
- W2: zweiter Winkel

## Patentansprüche

1. Embolieschutzvorrichtung (1) zur Zuführung in einen Aortenbogen, umfassend
eine Filtereinheit (3), einen Rahmen (5) und eine Zuführungseinheit (7), wobei die Filtereinheit (3) mit dem Rahmen (5) verbunden ist und der Rahmen (5) einen proximalen Bereich (9) aufweist, der eine Proximalform (11) umfasst, welche in einem Innenbereich des Rahmens (5) angeordnet ist und mit der Zuführungseinheit (7) verbunden ist, wobei die Proximalform (11) einen ersten Teil (13) und einen zweiten Teil (15) umfasst, wobei der zweite Teil (15) an einem Ende des ersten Teils (13) ausgebildet ist, **dadurch gekennzeichnet, dass** die Verbindung von Rahmen (5) und Filtereinheit (3) mittels eines Klebetunnels oder einer Klebetunnelverbindung realisiert ist.

2. Embolieschutzvorrichtung (1) nach Anspruch 1, **dadurch gekennzeichnet, dass** der erste Teil (13) der Proximalform (11) zur Ebene des Rahmens (5) einen ersten Winkel (W1) aufweist und der zweite Teil (15) zum ersten Teil (13) der Proximalform (11) einen zweiten Winkel (W2) aufweist.

3. Embolieschutzvorrichtung (1) zur Zuführung in einen Aortenbogen, umfassend
eine Filtereinheit (3), einen Rahmen (5) und eine Zuführungseinheit (7), wobei die Filtereinheit (3) mit dem Rahmen (5) verbunden ist und der Rahmen (5) einen proximalen Bereich (9) aufweist, der eine Proximalform (11) umfasst, welche in einem Innenbereich des Rahmens (5) angeordnet ist und mit der Zuführungseinheit (7) verbunden ist, wobei die Proximalform (11) einen ersten Teil (13) und einen zweiten Teil (15) umfasst, wobei der erste und zweite Teil (13, 15) derart zueinander angeordnet sind, dass sie einen Federmechanismus ausbilden, **dadurch gekennzeichnet, dass** die Verbindung von Rahmen (5) und Filtereinheit (3) mittels eines Klebetunnels oder einer Klebetunnelverbindung realisiert ist.

4. Embolieschutzvorrichtung (1) nach einem der Ansprüche 1-3, **dadurch gekennzeichnet, dass** die Proximalform (11) über die Zuführungseinheit (7) unter Spannung gesetzt werden kann.

5. Embolieschutzvorrichtung (1) nach einem der vorhergehenden Ansprüche, **dadurch gekennzeichnet, dass** die Proximalform (11) zwei Enden (17, 19) des Rahmens (5) umfasst, die sich parallel zueinander im Innenbereich des Rahmens (5) erstrecken.

6. Embolieschutzvorrichtung (1) nach einem der vorhergehenden Ansprüche, **dadurch gekennzeichnet, dass** der Rahmen (5) einen distalen Bereich (2) aufweist, der eine Distalform (4) umfasst, welche in einem Innenbereich des Rahmens (5) angeordnet ist.

7. Embolieschutzvorrichtung (1) nach Anspruch 6, **dadurch gekennzeichnet, dass** die Distalform (4) eine zum Inneren des Rahmens (5) hin gerichtete Einschnürung (12) aufweist.

8. Embolieschutzvorrichtung (1) nach einem der vorhergehenden Ansprüche, **dadurch gekennzeichnet, dass** die Filtereinheit (3) außerhalb des proximalen Bereichs (9), und/oder distalen Bereichs (2) nach Anspruch 6 und/oder 7, mit dem Rahmen (5) verbunden ist.

9. System aus einer Embolieschutzvorrichtung (1) nach einem der vorhergehenden Ansprüche und einer Formvorrichtung (31) zur Umformung der Embolieschutzvorrichtung (1) zum Einziehen in einen Schlauch (38), **dadurch gekennzeichnet, dass** ein Rahmen (5) mit einer daran angeordneten Filtereinheit (3) der Embolieschutzvorrichtung (1) von einem expandierten Zustand in einen gestreckten Zustand umgeformt wird, umfassend eine einseitig flache oder runde Öffnung (35), einen engsten Querschnitt (39) und eine gegenüberliegende runde Öffnung (37).

10. System nach Anspruch 9, wobei die flache oder runde Öffnung (35) der Formvorrichtung (31) so ausgebildet ist, dass die Proximalform (11) und/oder die Distalform (4) nach Anspruch 6 und/oder 7 des Rahmens (5) der Embolieschutzvorrichtung nach außen geklappt wird.

11. Verfahren zum Falten der Embolieschutzvorrichtung nach einem der Ansprüche 1-8 mittels der Formvorrichtung aus dem System nach einem der Ansprüche 9-10, umfassend Schieben (S1) des Rahmens (5) der Embolieschutzvorrichtung vor die flache oder runde Öffnung (35) der Formvorrichtung (31), wobei die Zuführungseinheit (7) durch die Formvorrichtung (31) hindurchgeführt wird,
Einziehen (S3) der Proximalform (11) in die Formvorrichtung (31), wobei die Proximalform (11) nach außen umgeklappt wird,
Einhaken (S4) der Distalform (4) über den äußeren Rand der Formvorrichtung (31), wobei durch weiteres Ziehen die Distalform (4) nach außen umgeklappt und in die Formvorrichtung (31) eingezogen wird.

12. Verfahren nach Anspruch 11, wobei die umgeklappte Proximalform (11) eine Vorspannung auf den Rahmen (5) überträgt, die im Wesentlichen gleich ist mit der Spannung, die daraus resultiert, dass die gebogene Proximalform (11) begradigt wird.

## Claims

1. Embolism protection device (1) for delivery into an aortic arch, comprising
a filter unit (3), a frame (5) and a supply unit (7), wherein the filter unit (3) is connected to the frame (5), and the frame (5) has a proximal region (9), which comprises a proximal mould (11), which is arranged in an inner region of the frame (5) and is connected to the supply unit (7), the proximal mould (11) comprising a first part (13) and a second part (15), the second part (15) being formed at one end of the first part (13), **characterised in that** the connection of the frame (5) and the filter unit (3) is realised by means of an adhesive tunnel or an adhesive tunnel connection.

2. Embolism protection device (1) according to claim 1, **characterised in that** the first part (13) of the proximal mould (11) has a first angle (W1) with respect to the plane of the frame (5), and the second part (15) has a second angle (W2) with respect to the first part (13) of the proximal mould (11).

3. Embolism protection device (1) for delivery into an aortic arch, comprising
a filter unit (3), a frame (5) and a supply unit (7), wherein the filter unit (3) is connected to the frame (5), and the frame (5) has a proximal region (9), which comprises a proximal mould (11), which is arranged in an inner region of the frame (5) and is connected to the supply unit (7), the proximal mould (11) comprising a first part (13) and a second part (15), the first and second parts (13, 15) being arranged with respect to one another in such a way that they form a spring mechanism, **characterised in that** the connection of the frame (5) and the filter unit (3) is realised by means of an adhesive tunnel or an adhesive tunnel connection.

4. Embolism protection device (1) according to one of claims 1-3, **characterised in that** the proximal mould (11) can be energised via the supply unit (7).

5. Embolism protection device (1) according to any of the preceding claims, **characterised in that** the proximal mould (11) comprises two ends (17, 19) of the frame (5), which extend parallel to one another in the inner region of the frame (5).

6. Embolism protection device (1) according to any of the preceding claims, **characterised in that** the frame (5) has a distal region (2), which comprises a distal mould (4), which is arranged in an inner region of the frame (5).

7. Embolism protection device (1) according to claim 6, **characterised in that** the distal mould (4) has a constriction (12) directed towards the interior of the frame (5).

8. Embolism protection device (1) according to any of the preceding claims, **characterised in that** the filter unit (3) is connected to the frame (5) outside the proximal region (9) and/or distal region (2) according to claim 6 and/or 7.

9. System comprising an embolism protection device (1) according to any of the preceding claims and a moulding device (31) for forming the embolism protection device (1) for drawing into a hose (38), **characterised in that** a frame (5) with a filter unit (3) of the embolism protection device (1) arranged thereon is formed from an expanded state into a stretched state, comprising a flat or round opening (35) on one side, a narrowest cross-section (39) and an opposite round opening (37).

10. System according to claim 9, wherein the flat or round opening (35) of the moulding device (31) is designed such that the proximal mould (11) and/or the distal mould (4) according to claim 6 and/or 7 of the frame (5) of the embolism protection device is folded outwards.

11. Method for folding the embolism protection device according to one of claims 1-8 by means of the moulding device from the system according to one of claims 9-10, comprising
pushing (S1) the frame (5) of the embolism protection device in front of the flat or round opening (35) of the moulding device (31), wherein the supply unit (7) is guided through the moulding device (31),
drawing (S3) the proximal mould (11) into the moulding device (31), wherein the proximal mould (11) is folded outwards,
hooking (S4) the distal mould (4) over the outer edge of the moulding device (31), wherein the distal mould (4) is folded outwards and drawn into the moulding device (31) by further pulling.

12. Method according to claim 11, wherein the folded proximal mould (11) transfers a preload to the frame (5) that is substantially equal to the load resulting from straightening the bent proximal mould (11).

## Revendications

1. Dispositif de protection embolique (1) destiné à être introduit dans une crosse aortique, comprenant
une unité de filtration (3), un cadre (5) et une unité d'introduction (7), ladite unité de filtration (3) étant reliée au cadre (5) et ledit cadre (5) présentant une zone proximale (9) qui comprend un moule proximal (11), lequel est disposé dans une zone interne du cadre (5) et qui est relié à l'unité d'introduction (7), ledit moule proximal (11) comprenant une première partie (13) et une seconde partie (15), ladite seconde partie (15) étant formée au niveau d'une extrémité de la première partie (13), **caractérisé en ce que** le raccordement du cadre (5) et de l'unité de filtration (3) est réalisé au moyen d'un tunnel adhésif ou d'un raccord de tunnel adhésif.

2. Dispositif de protection embolique (1) selon la revendication 1, **caractérisé en ce que** la première partie (13) de le moule proximal (11) présente un premier angle (W1) par rapport au plan du cadre (5) et la seconde partie (15) présente un second angle (W2) par rapport à la première partie (13) du moule proximal (11).

3. Dispositif de protection embolique (1) destiné à être introduit dans une crosse aortique, comprenant
une unité de filtration (3), un cadre (5) et une unité d'introduction (7), ladite unité de filtration (3) étant reliée au cadre (5) et ledit cadre (5) présentant une zone proximale (9) qui comprend un moule proximal (11), lequel est disposé dans une zone interne du cadre (5) et est relié à l'unité d'introduction (7), ledit moule proximal (11) comprenant une première partie (13) et une seconde partie (15), lesdites première et seconde parties (13, 15) étant disposées l'une par rapport à l'autre de manière à former un mécanisme à ressort, **caractérisé en ce que** le raccordement du cadre (5) et de l'unité de filtration (3) est réalisé au moyen d'un tunnel adhésif ou d'un raccord de tunnel adhésif.

4. Dispositif de protection embolique (1) selon l'une des revendications 1 à 3, **caractérisé en ce que** le moule proximal (11) peut être mis sous contrainte par l'intermédiaire de l'unité d'introduction (7).

5. Dispositif de protection embolique (1) selon l'une des revendications précédentes, **caractérisé en ce que** le moule proximal (11) comprend deux extrémités (17, 19) du cadre (5) qui s'étendent parallèlement l'une à l'autre dans la zone interne du cadre (5).

6. Dispositif de protection embolique (1) selon l'une des revendications précédentes, **caractérisé en ce que** le cadre (5) présente une zone distale (2) qui comprend un moule distal (4) qui est disposé dans une zone interne du cadre (5).

7. Dispositif de protection embolique (1) selon la revendication 6, **caractérisé en ce que** le moule distal (4) présente un rétrécissement (12) dirigé vers l'intérieur du cadre (5).

8. Dispositif de protection embolique (1) selon l'une des revendications précédentes, **caractérisé en ce que** l'unité de filtration (3) est reliée au cadre (5) en dehors de la zone proximale (9) et/ou de la zone distale (2) selon la revendication 6 et/ou 7.

9. Système constitué d'un dispositif de protection embolique (1) selon l'une des revendications précédentes et d'un dispositif de moulage (31) destiné à transformer le dispositif de protection embolique (1) en un tuyau (38), **caractérisé en ce qu'**un cadre (5) avec une unité de filtration (3) du dispositif de protection embolique (1) disposée sur celui-ci est transformé d'un état expansé en un état étiré, comprenant une ouverture (35) plate ou ronde d'un côté, la section transversale (39) la plus étroite et une ouverture ronde (37) opposée.

10. Système selon la revendication 9, ladite ouverture (35) plate ou ronde du dispositif de moulage (31) étant conçue de manière à ce que le moule proximal (11) et/ou le moule distal (4) selon la revendication 6 et/ou 7 du cadre (5) du dispositif de protection embolique soient pliés vers l'extérieur.

11. Procédé de pliage du dispositif de protection embolique selon l'une des revendications 1 à 8 au moyen du dispositif de moulage du système selon l'une des revendications 9 à 10, comprenant
la poussée (S1) du cadre (5) du dispositif de protection embolique devant l'ouverture (35) plate ou ronde du dispositif de moulage (31), ladite unité d'introduction (7) étant guidée à travers le dispositif de moulage (31),
la rétractation (S3) du moule proximal (11) dans le dispositif de moulage (31), ledit moule proximal (11) étant replié vers l'extérieur,
l'accrochage (S4) du moule distal (4) sur le bord externe du dispositif de moulage (31), par une traction supplémentaire ledit moule distal (4) étant replié vers l'extérieur et rentré dans le dispositif de moulage (31).

12. Procédé selon la revendication 11, ledit moule proximal replié (11) transmettant au cadre (5) une précontrainte qui est sensiblement égale à la contrainte résultant du redressement du moule proximal courbé (11).
